# EUROPEAN PATENT SPECIFICATION

(11) **EP 3 015 445 B1**
(45) Date of publication and mention of the grant of the patent: **26.08.2020**
(21) Application number: 14191061.2
(22) Date of filing: 30.10.2014
(51) Int. Cl.: C07C 1/20, C07C 15/02, C07C 2/86, C07C 15/08, C07C 6/12, C10G 3/00, C10G 29/20, B01J 29/18, B01J 29/40, B01J 29/44, B01J 29/70

(54) **A method for producing an aromatic hydrocarbon with an oxygenate as raw material**
Verfahren zur Herstellung eines aromatischen Kohlenwasserstoffs mit einem Oxygenat als Rohmaterial
Procédé de production d'un hydrocarbure aromatique avec un composé oxygéné en tant que matière première

(43) Date of publication of application: 04.05.2016
(73) Proprietor: China Petroleum & Chemical Corporation, Beijing 100728 (CN); Shanghai Research Institute of Petrochemical Technology SINOPEC, Pudong New Area, Shanghai 201208 (CN)
(72) Inventor: Wang, Zheming, 201208 Shanghei (CN); Chen, Xiqiang, 201208 Shanghei (CN); Xu, Feng, 201208 Shanghei (CN); Yang, Weimin, 201208 Shanghei (CN); Xiao, Jingxian, 201208 Shanghei (CN)
(74) Representative: Hoffmann Eitle

(56) References cited:
- CN-A- 101 671 226
- CN-B- 101 607 864
- US-A1- 2013 165 725

## Description

### Technical field

The present invention relates to a method for producing an aromatic hydrocarbon with an oxygenate as raw material.

### Background technology

Aromatic hydrocarbon (wherein benzene, methylbenzene and dimethylbenzene(i.e. xylene) are referred to be B, T and X respectively and they are jointly called as BTX) is an important basic organic chemical material. Around 90% of aromatic hydrocarbons all over the world comes from the catalytic reforming process of petroleum as raw material and from the gasoline as a byproduct from the steam cracking.Only 10% of the total yield of aromatic hydrocarbons comes from the coal route. With the increasing depletion of petroleum resources and thus the high price thereof, the energy and chemical industry with mainly a petroleum route faces unprecedented serious challenges.

With the development of natural gas and shale gas in North America and Middle East, a large amount of light hydrocarbons are produced as a byproduct. The light hydrocarbon produced by shale gas as byproduct replaces a part of naphtha for steam cracking, and thus a trend of conversion to a light fraction appears in the raw material for steam cracking. In the future, a reduction trend may be seen in the yield of aromatic hydrocarbons from steam cracking byproducts, which results in a shortage trend of the yield of aromatic hydrocarbons all over the world in the future. Thus, the development of a new technology using an oxygenate including methanol as raw material for a partial substitute of the production of aromatic hydrocarbon with petroleum has a great potential.

U.S. patent, US3931731 reported a method for the production of gasoline with methanol as raw material. Although a considerable number of aromatic hydrocarbons are present in gasoline, the process takes the production of gasoline liquid fuel with a high octane number as a main target and the product further contains a large amount of isoparaffin components having high octane numbers. Thus, the technology of producing gasoline with oxygenate(s) as raw material(s) has a technical problem of a low total yield of aromatic hydrocarbons.

Chinese patent CN101244969B reported a C₁-C₁₂ hydrocarbons or a methanol aromatization and catalyst regeneration device. The method does not relate to the conversion of non-hydrocarbons in the reaction product, specific reaction or separation procedures. The added value of non-hydrocarbons in the product is relatively low. Moreover, the production of a large mount of non-hydrocarbons in the product will reduce the efficiency of aromatization of raw material and increase the cost during aromatization.

Chinese patent CN101671226 reported conducting aromatization reaction in an aromatization reactor with a mixture of methanol and one or more of C₁-C₁₂ hydrocarbons. The method only takes into consideration of a one-time conversion of methanol and C₁-C₁₂ hydrocarbons and does not relate to a cycle conversion of non-hydrocarbons to aromatic hydrocarbons in the reaction product. Thus, the process has a problem of a low yield of aromatic hydrocarbon. The research shows that the reaction temperature of methane aromatization is up to 700°C, but the conversion rate of methane is less than 20% and the yield of aromatic hydrocarbons is only around 10%. The highest reaction temperature of aromatization in the process is only 650 °C. Thus, if the components of C₁-C₁₂ hydrocarbons contain methane of lower activity, the presence of methane or the accumulation thereof in cycle flow may reduce the utilization efficiency of the reactor.

Chinese patent CN101820919B reported a procedure of producing a dimethylbenzene product with methanol or an oxygenate. In the procedure, B and T in the liquid phase aromatic hydrocarbon product are separated one by one, as well as non-aromatic hydrocarbons having more than 6 carbon numbers are also separated. In the aromatic hydrocarbon mixture, non-aromatic hydrocarbons and aromatic hydrocarbons with the same number of carbon atoms have extremely close boiling points and it is very difficult to separate them. The current technique of separating aromatic hydrocarbon usually achieves the separation of non-aromatic hydrocarbons from aromatic hydrocarbons by a manner of solvent extraction of a mixed hydrocarbons flow containing benzene, methylbenzene and dimethylbenzene light aromatic hydrocarbon and then separates benzene, methylbenzene, dimethylbenzene and C₉⁺ aromatic hydrocarbon one by one. In the procedure disclosed in Chinese patent CN101820919B, non-aromatic hydrocarbons having no more than 6 carbon numbers are subjected to a cycle conversion to aromatic hydrocarbon. However, non-aromatic hydrocarbons having more than 6 carbon numbers are not subjected to a cycle conversion to aromatic hydrocarbon. Thus, a problem of a low aromatic hydrocarbon yield is present during the process. As compared to the direction utilization of a hydrocarbons mixture containing B and T, the energy consumption during the separation process of liquid phase aromatic hydrocarbon from non-aromatic hydrocarbon in Chinese patent CN101820919B will be higher. Moreover, in said procedure, simply only H₂ and CH₄ are removed from the gas phase components thereof. Oxygenates such as CO, CO₂, formaldehyde, formic acid and acetic acid are produced inevitably during the process of producing aromatic hydrocarbon with oxygenates. These components cannot be further converted to aromatic hydrocarbon. Without removal, they will be accumulated in the reaction system and thus affect the efficiency of the reactor.

Chinese patent CN101607864B reported a method of increasing the yield of dimethylbenzene by adding benzene or methylbenzene to the aromatization system of oxygenate. In the product of producing aromatic hydrocarbon with oxygenate, there are a large amount of non-aromatic hydrocarbons, unconverted oxygenates and intermediate product of oxygenate in addition to aromatic hydrocarbon products. These non-aromatic hydrocarbon products have a large amount of components. If separating them or selling them as mixture, their added values are relatively low. By adding benzene or methylbenzene components from the reaction products or from outside to the aromatization process, the yield of dimethylbenzene product may be increased via the alkylation of methanol. However, the method requires relatively high energy consumption for separating benzene and methylbenzene respectively, which will necessarily increase the production cost of aromatic hydrocarbon. In addition, the aromatization catalyst used in the method uses a molecular sieve catalyst upon silanization and metal modification. Although the silanization modification will improve the shape selectivity of the catalyst to a certain degree, it will cause the blockage of pores and reduction of catalyst activity.

Chinese patent application CN1880288A reported a process of methanol conversion for preparing aromatic hydrocarbons and catalyst. In the technique of preparing aromatic hydrocarbons with methanol disclosed by the above patent, two fixed-bed reactors in series are used; after the reactant, methanol, enters the first section of the reactor for reaction, the first section of the gas phase product continues to enter the second section of reactor for reaction, aromatic hydrocarbon and non-aromatic hydrocarbon are obtained upon separation of the first and second sections of liquid phase products. The process of producing aromatic hydrocarbons with oxygenates is a process of strong exothermicity and relatively rapid deactivation due to carbon deposition. The fixed-bed reactor has a difficulty of heat transfer and heat removal and has a problem of being difficult to control the temperature stably. In the process, only the gas phase components in the byproducts of the first reactor are used for aromatization thereof for conversation to aromatic hydrocarbons. The non-aromatic hydrocarbons as the byproducts in the second reactor are not further subjected to cycle conversion to aromatic hydrocarbon. Thus, the process has a technical problem of a low total yield of aromatic hydrocarbon.

U.S. patent application US20100185033A1, reported a method of producing aromatic hydrocarbons with aliphatic alcohols having carbon numbers between 1 and 10 as raw material. The catalyst is a molecular sieve catalyst loaded with 0.0001 to 20 % of La and 0.0001-20 % of M metal, wherein M is selected from at least one of molybdenum (Mo), cerium (Ce), or caesium (Cs) and the zoelite is selected from ZSM-5, ZSM-11, ZSM-23, ZSM-48 and ZSM-57. The reaction process conditions are a temperature of 250 to 750°C, a pressure of 0 to 3MPa, a raw material space velocity of 0.1 to 500h⁻¹. The method does not relate to a step of producing aromatic hydrocarbon by a further cycle conversion of a byproduct, non-aromatic hydrocarbons. Thus, the process has a problem of a low total yield of aromatic hydrocarbons.

U.S. patent US6489528B2 reported a method of producing aromatic hydrocarbon with methanol or dimethyl ether as raw material and two types of molecular sieve catalysts, wherein one of them is a silicoaluminophosphate molecular sieve and another one is a ZSM-5 molecular sieve catalyst which contains a metal Zn and an element from Group IIIA or Group VIB. The method does not mention specific reaction and separation procedures and there is no further utilization solution of non-aromatic hydrocarbon components in the product.

In addition to aromatic hydrocarbon products, there are a large amount of non-aromatic hydrocarbon hydrocarbons components, a small amount of unconverted oxygenate materials and other intermediate oxygenate components as byproducts in the products from the production of aromatic hydrocarbons with oxygenates. These components are very complicated. The economy of using them alone upon separation is poor. As fuel gas, their added values are also very low. If such part of components can be converted to aromatic hydrocarbons, a total yield of aromatic hydrocarbons in the production of aromatic hydrocarbons with oxygenates can be notably improved, the production cost of aromatic hydrocarbons can be reduced and a notable economic benefit is produced.

In the components of aromatic hydrocarbons, a light aromatic hydrocarbon-BTX is an aromatic hydrocarbon product having the most wide value and use. In the aromatic hydrocarbon components, dimethylbenzene is a product having a relatively wide use and a relatively high added value. In the aromatic hydrocarbon products, the paths for using methylbenzene directly are very limited. Its main use is converting methylbenzene to dimethylbenzene product which is in great demand and has a relatively high added value through selective disproportionation of methylbenzene or a transalkylation process with a C₉ component.

Introducing non-metal oxides and metal oxides, especially non-metal oxides, for modification is reported in the prior art. However, the presence of these components may cause a blockage of molecular sieve pores, a failure to achieve the optimum modification effect and a reduction of aromatization yield of catalyst.

To sum up, the technical problem of a low total yield of aromatic hydrocarbons and a high energy consumption is present during the process of producing aromatic hydrocarbons with oxygenates as raw material in the prior art.

US 2013/0165725 describes a process for the preparation of an aromatic product comprising xylene, wherein the process comprises converting an oxygenate feedstock in a specific reaction zone to obtain a conversion effluent comprising benzene, toluene, xylene and olefins, separating at least a portion of the benzene and toluene from the conversion effluent to form an aromatics-containing stream, separating the olefins from the conversion effluent, separating xylene from the conversion effluent to produce a xylene product stream and recycling at least a portion of the aromatics-containing stream to the first step mentioned above.

### The contents of the invention

The technical problem to be solved by the present invention is providing a method for producing an aromatic hydrocarbon with an oxygenate as raw material. The method has advantages of a high yield of an aromatic hydrocarbon and a low energy consumption.

The present invention relates to a method for producing an aromatic hydrocarbon with an oxygenate as raw material, comprising
i) reacting an oxygenate in at least one aromatization reactor to obtain an aromatization reaction product;
ii) separating the aromatization reaction product through a separation unit A to obtain a gas phase hydrocarbons flow X and a liquid phase hydrocarbons flow Y;
iii) after removing gas and/or a part of the oxygenate from the gas phase hydrocarbons flow X through a separation unit B, a reaction is conducted in another aromatization reactor and a separation is conducted through a separation unit A, to obtain a flow X2 containing a non-aromatic hydrocarbon and a flow X3 containing an aromatic hydrocarbon;
iv) a mixed hydrocarbons flow M of an aromatic hydrocarbon having less than or equal to 7 carbon numbers and a flow N of the residual hydrocarbons is obtained by a non-precise rectification of the liquid phase hydrocarbons flow Y in a separation unit C, optionally after combining said flow Y with the flow X3;
v) separating the flow N of the residual hydrocarbons through a separation unit D, to obtain a flow K containing a non-aromatic hydrocarbon, a C₈ aromatic hydrocarbon flow J and a C₉⁺ aromatic hydrocarbon flow L;
vi) returning
   a) the flow X2 containing a non-aromatic hydrocarbon, and
   b) the mixed hydrocarbons flow M of an aromatic hydrocarbon having less than or equal to 7 carbon numbers and/or a part or all of the flow K containing a non-aromatic hydrocarbon, and optionally
   c) an additional C₂⁺ hydrocarbons flow,
   to the above oxygenate; or returning
   a) the flow X2 containing a non-aromatic hydrocarbon, and
   b) a mixed hydrocarbons flow M of an aromatic hydrocarbon having less than or equal to 7 carbon numbers and/or a part or all of the flow K containing a non-aromatic hydrocarbon,
   to the aromatization reactor in iii);
vii) optionally, reacting the C₉⁺ aromatic hydrocarbons flow L in at least one reactor selected from a transalkylation reactor and a dealkylation reactor to obtain a C aromatic hydrocarbon flow L1.

Therein the oxygenate is preferably selected from at least one of methanol and dimethyl ether. The separation unit A preferably comprises operation units such as quenching, alkaline washing and/or water washing. The separation unit B preferably comprises at least one of separation manners such as pressure swing adsorption, rectification and adsorption. The separation unit C is a non-precise rectification separation unit, by which a liquid phase hydrocarbons flow Y containing a non-aromatic hydrocarbon and an aromatic hydrocarbon can be separated to a mixed hydrocarbons flow M of an aromatic hydrocarbon having less than or equal to 7 carbon numbers and a mixed hydrocarbons flow N of the residual hydrocarbons and non-aromatic hydrocarbons. This is different from the precise rectification separation of aromatic hydrocarbon in the prior art. The precise rectification separation of an aromatic hydrocarbon in the prior art can separate non-hydrocarbon components of the flow Y from aromatic hydrocarbon components completely by solvent extraction and further separate the aromatic hydrocarbon mixture thereof to benzene, methylbenzene, C₈ aromatic hydrocarbon and C₉⁺ aromatic hydrocarbon. The separation unit D preferably comprises at least one of rectification and solvent extraction rectification.

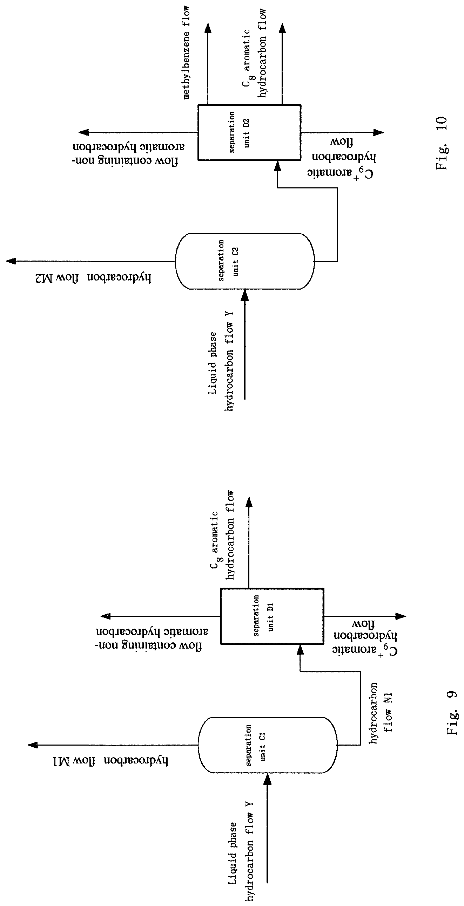

In the above method of the present invention, the liquid phase hydrocarbons flow Y is preferably separated according to the following two separation manners:
1) flow Y enters a separation unit C1 and is separated by a non-precise rectification to obtain a mixed hydrocarbons flow M1 of aromatic hydrocarbons having less than or equal to 6 carbon numbers and a hydrocarbons flow N1 having more than 6 carbon numbers, and the hydrocarbons flow N1 enters a separation unit D1 to be able to obtain a C₈ aromatic hydrocarbon flow and a C₉⁺ aromatic hydrocarbon flow, for example, with reference to Figure 9, the separation flow chart;
2) flow Y enters a separation unit C2 and is separated by a non-precise rectification to obtain a mixed hydrocarbons flow M2 of aromatic hydrocarbons having less than or equal to 7 carbon numbers and a hydrocarbons flow N2 having more than 7 carbon numbers, and the hydrocarbons flow N2 enters a separation unit D2 to be able to obtain a C₈ aromatic hydrocarbon flow and a C₉⁺ aromatic hydrocarbon flow, with reference to Figure 10, the separation flow chart.

In the method of the present invention, a part or all of the flow containing non-aromatic hydrocarbons and the flow of the oxygenate may come into contact with a catalyst for reaction in the same aromatization reactor or by entering different aromatization reactors respectively. The method of the present invention preferably comprises at least one reactor selected from the group consisting of a transalkylation reactor and a dealkylation reactor for converting a C₉⁺ aromatic hydrocarbon flow L in the product of aromatic hydrocarbon to dimethylbenzene; the reaction conditions for said transalkylation reactor are preferably a temperature of 350 to 550°C, a reaction pressure of 0.1 to 5.0MPa, a molar ratio of hydrogen/hydrocarbon of 1: 1 to 200: 1, a weight space velocity of raw material(s) of 0.1 to 15 h⁻¹, preferably 0.1 to 5 h⁻¹; the reaction conditions of said dealkylation reactor are: a reaction temperature of 300 to 800°C, a molar ratio of hydrogen/hydrocarbon of 0.1 to 200: 1 and a weight space velocity of the hydrocarbons of 0.5 to 10 h⁻¹.

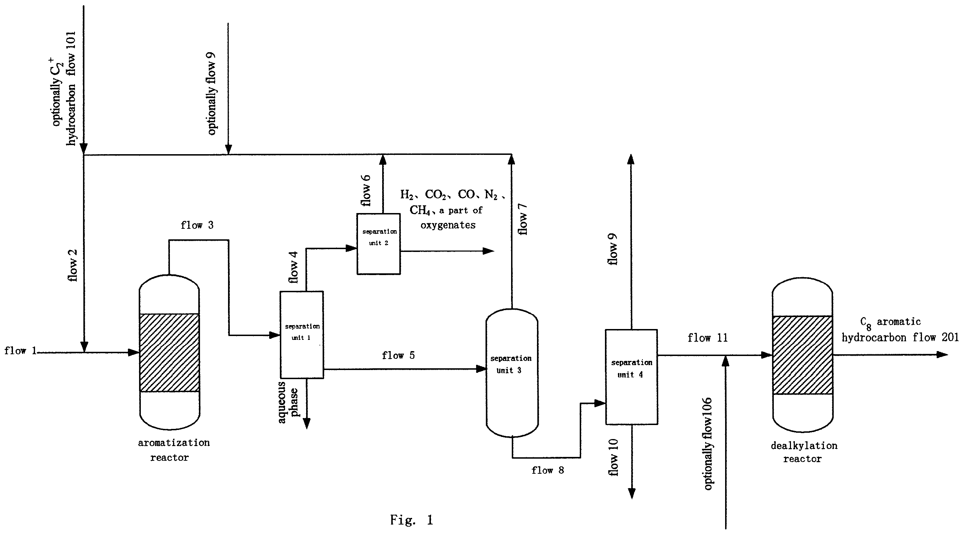

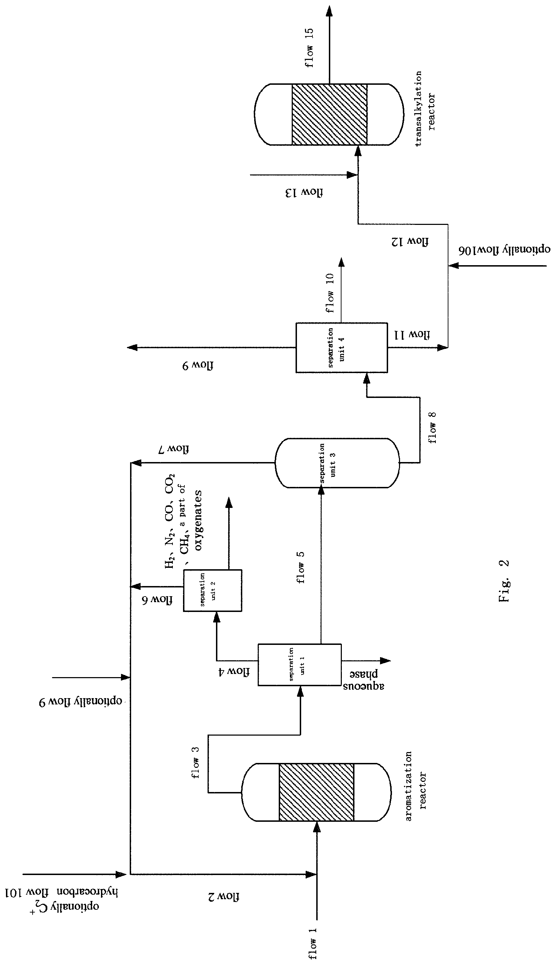

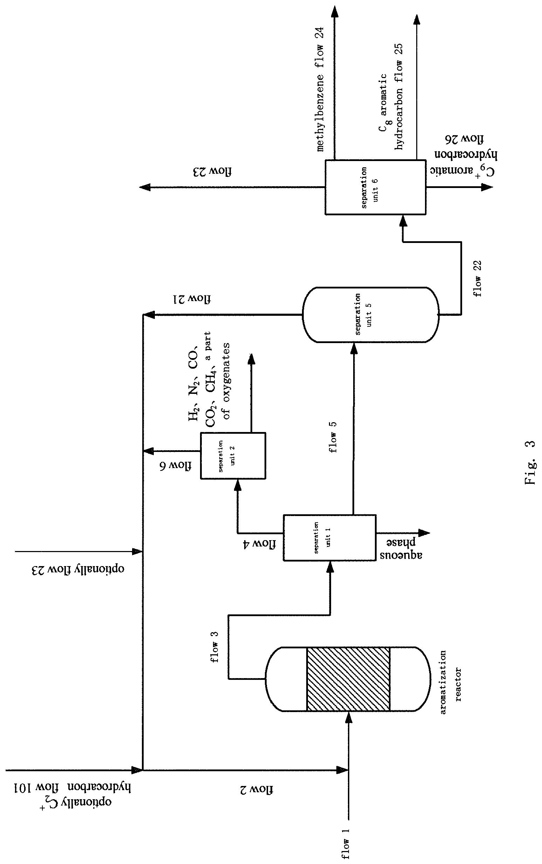

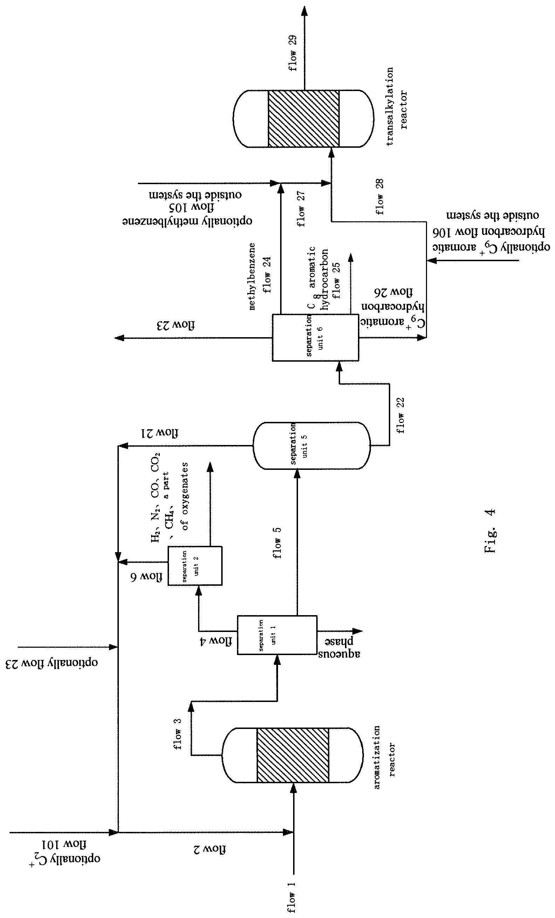

When the method of the present invention contains one aromatization reactor, the method of the present invention is conducted according to the following steps:
a) under the process conditions of a temperature of 400 to 550°C, a pressure of 0.01 to 2.0MPa and a weight space velocity of raw material(s) of 0.1 to 4 h⁻¹, contacting a flow 1 of oxygenate(s) with a catalyst for reaction in an aromatization reactor to obtain a hydrocarbons flow 3;
b) removing CO₂ and a part of oxygenate(s) from said hydrocarbons flow 3 through a separation unit 1 to obtain a gas phase non-aromatic hydrocarbons flow 4, a liquid phase hydrocarbons flow 5 containing an aromatic hydrocarbon and an aqueous phase;
c) removing gas, such as, inorganic gas comprising H₂, CO, CO₂, N₂ and the like, CH₄ and a part of oxygenate(s) from said gas phase non-aromatic hydrocarbons flow 4 through a separation unit 2 to obtain a C₂⁺ hydrocarbons flow 6;
d) further separating the liquid phase hydrocarbons flow 5 containing an aromatic hydrocarbon according to one of the following four manners, and reacting:
   d1) for example, with reference to Figure 1, subjecting the liquid phase hydrocarbons flow 5 containing an aromatic hydrocarbon to non-precise rectification through a separation unit 3 to obtain a hydrocarbon flow 7 of aromatic hydrocarbons having less than or equal to 7 carbon numbers and a hydrocarbon flow 8 of aromatic hydrocarbons having more than 7 carbon numbers, and separating said hydrocarbons flow 8 through a separation unit 4 to obtain a hydrocarbons flow 9, a flow 10 containing C₈ aromatic hydrocarbon and a C₉⁺ aromatic hydrocarbon flow 11, and reacting said flow 11 in a dealkylation reactor to obtain a C₈ aromatic hydrocarbon flow 201;
      obtaining a hydrocarbons flow 2 of aromatic hydrocarbons having less than or equal to 7 carbon numbers from the hydrocarbons flow 7 and a part or all of the C₂⁺ hydrocarbons flow 6, wherein said hydrocarbons flow 2 further optionally comprises a part or all of at least one selected from the hydrocarbons flow 9 and a C₂⁺ hydrocarbons flow 101 outside the reaction-separation system; and returning said hydrocarbons flow 2 to the oxygenate(s) flow 1 for further reaction;
   d2) for example, with reference to Figure 2, subjecting the liquid phase hydrocarbons flow 5 containing an aromatic hydrocarbon to a non-precise rectification through a separation unit 3 to obtain a hydrocarbon flow 7 of aromatic hydrocarbons having less than or equal to 7 carbon numbers and a hydrocarbons flow 8 of aromatic hydrocarbons having more than 7 carbon numbers, and obtaining a hydrocarbons flow 9 containing non-aromatic hydrocarbons, a flow 10 containing C₈ aromatic hydrocarbons and a C₉⁺ aromatic hydrocarbon flow 11 from said hydrocarbons flow 8 through a separation unit 4;
      obtaining a hydrocarbon flow 15 containing dimethylbenzene from a C₉⁺ aromatic hydrocarbon flow 12 and a methylbenzene flow 13 outside the reaction-separation system through a transalkylation reactor, wherein the C₉⁺ aromatic hydrocarbon flow 12 is selected from one of a part or all of a C₉⁺ aromatic hydrocarbon flow 11 or a mixture of a part or all of the C₉⁺ aromatic hydrocarbon flow 11 and a C₉⁺ aromatic hydrocarbon flow 106 outside the reaction-separation system;
      obtaining a hydrocarbons flow 2 of aromatic hydrocarbon having less than or equal to 7 carbon numbers from the hydrocarbons flow 7 and a part or all of a C₂⁺ hydrocarbons flow 6, wherein said hydrocarbons flow 2 further optionally comprises a part or all of at least one selected from the hydrocarbons flow 9 and a C₂⁺ hydrocarbons flow 101 outside the reaction-separation system; and returning said hydrocarbons flow 2 to the oxygenate(s) flow 1 for further reaction;
   d3) for example, with reference to Figure 3, subjecting the liquid phase hydrocarbons flow 5 containing an aromatic hydrocarbon to a non-precise rectification through a separation unit 5 to obtain a hydrocarbon flow 21 of aromatic hydrocarbon having less than or equal to 6 carbon numbers and a hydrocarbons flow 22 of aromatic hydrocarbon having more than 6 carbon numbers, and obtaining a hydrocarbons flow 23 containing non-aromatic hydrocarbons, a methylbenzene flow 24, a flow 25 containing C₈ aromatic hydrocarbons and a C₉⁺ aromatic hydrocarbon flow 26 from said flow 22 through a separation unit 6;
      obtaining a hydrocarbons flow 2 of aromatic hydrocarbons having less than or equal to 7 carbon numbers from a part or all of the hydrocarbons flow 6 and the hydrocarbons flow 21, wherein said hydrocarbons flow 2 further optionally comprises a part or all of at least one selected from the hydrocarbons flow 23 and a C₂⁺ hydrocarbons flow 101 outside the reaction-separation system; and returning said hydrocarbons flow 2 to the oxygenate flow 1 for further reaction;
   d4) for example, with reference to Figure 4, subjecting the liquid phase hydrocarbons flow 5 containing an aromatic hydrocarbon to a non-precise rectification through a separation unit 5 to obtain a hydrocarbons flow 21 of aromatic hydrocarbons having less than or equal to 6 carbon numbers and a hydrocarbons flow 22 of aromatic hydrocarbons having more than 6 carbon numbers, and obtaining a hydrocarbon flow 23 containing non-aromatic hydrocarbons, a methylbenzene flow 24, a flow 25 containing Cg aromatic hydrocarbons and a C₉⁺ aromatic hydrocarbon flow 26 from said flow 22 through a separation unit 6;
      contacting a methylbenzene flow 27 and a C₉⁺ aromatic hydrocarbon flow 28 with a catalyst in a transalkylation reactor to obtain a flow 29 containing dimethylbenzene, wherein said flow 27 is selected from one of a part or all of the methylbenzene flow 24 or a mixed flow of a part or all of the flow 24 and a methylbenzene flow 105 outside the reaction-separation system, and said C₉⁺ aromatic hydrocarbon flow 28 is selected from one of a part or all of the flow 26 or a mixed flow of a part or all of the flow 26 and a C₉⁺ aromatic hydrocarbon flow 106 outside the reaction-separation system;
      obtaining a hydrocarbons flow 2 of aromatic hydrocarbons having less than or equal to 7 carbon numbers from a part or all of the hydrocarbon flow 6 and the hydrocarbons flow 21, wherein said hydrocarbons flow 2 further optionally comprises a part or all of at least one selected from the flow 23 and a C₂⁺ hydrocarbons flow 101 outside the reaction-separation system; and returning said hydrocarbons flow 2 to the oxygenate(s) flow 1 for further reaction.

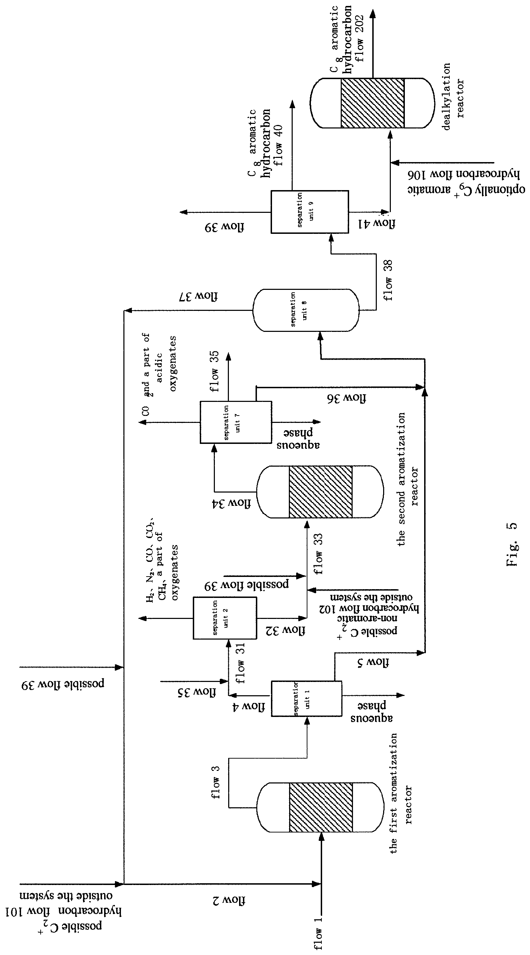

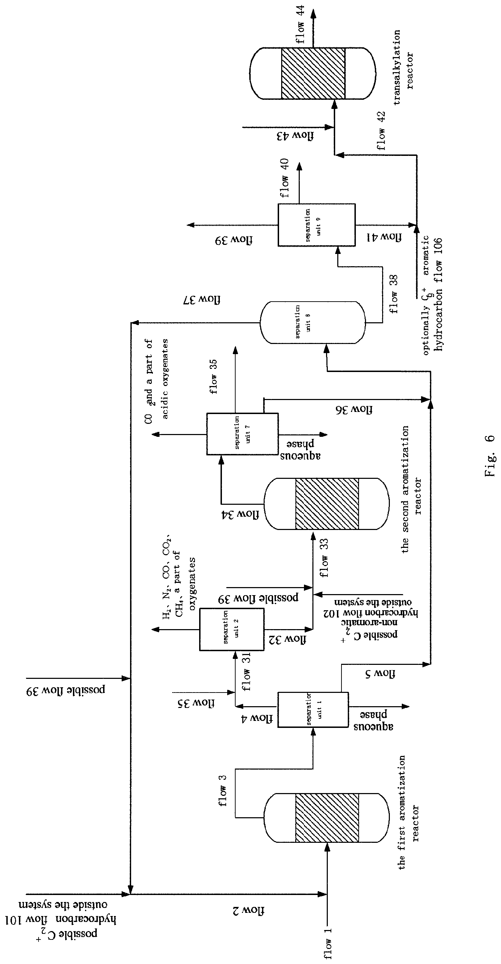

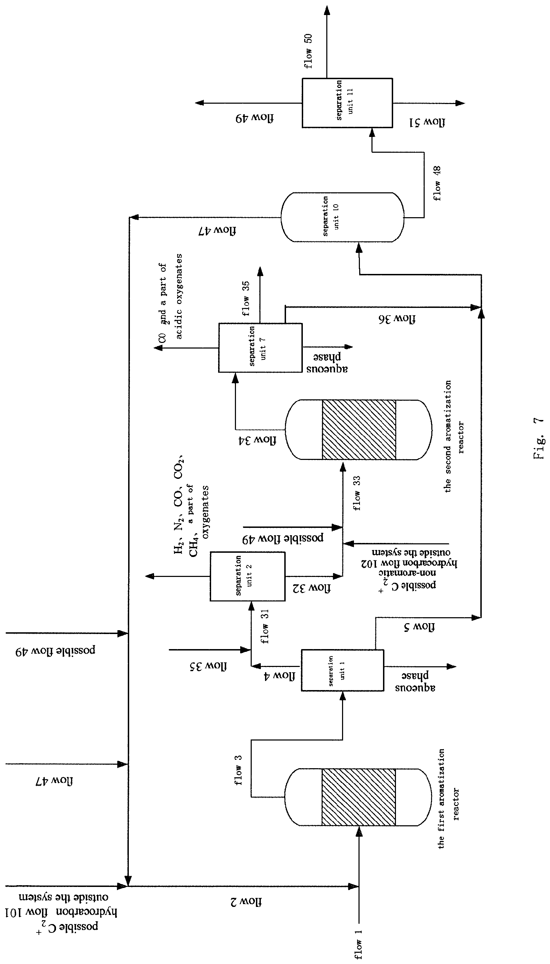

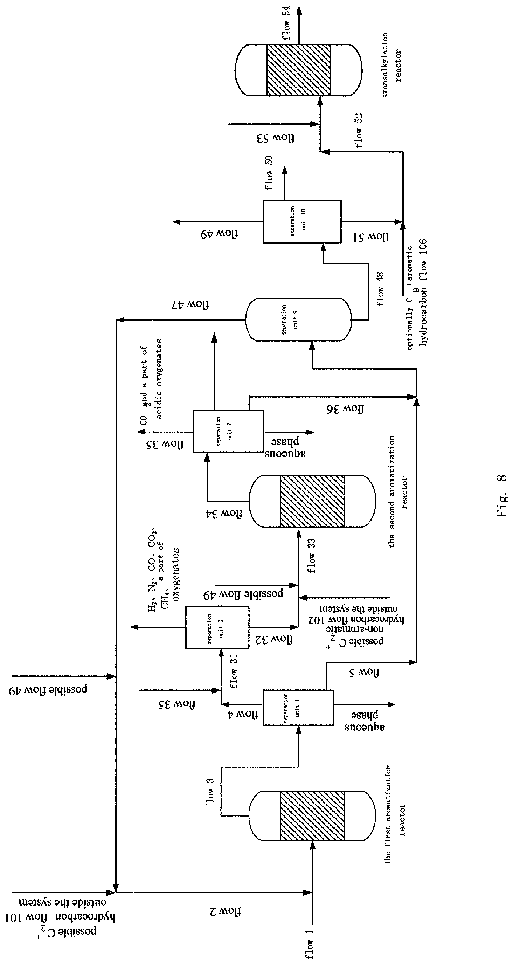

   When the method of the present invention contains two aromatization reactors, the method of the present invention is conducted according to the following steps.
h) under the process conditions of a temperature of 400 to 550°C, a reaction pressure of 0.01 to 2.0MPa and a weight space velocity of raw material(s) of 0.1 to 4 h⁻¹, contacting a flow 1 of oxygenate(s) with a catalyst for reaction in a first aromatization reactor to obtain a hydrocarbons flow 3;
i) removing CO₂ and a part of acidic oxygenate to obtain a gas phase non-aromatic hydrocarbon flow 4, a liquid phase hydrocarbons flow 5 containing aromatic hydrocarbons and an aqueous phase from a flow 3 through a separation unit 1;
j) removing gas, such as, inorganic gas comprising H₂, CO, CO₂, N₂ and the like, CH₄ and a part of oxygenate(s) from said hydrocarbons flow 31 through a separation unit 2 to obtain a C₂⁺ non-aromatic hydrocarbons flow 32, wherein the hydrocarbons flow 31 is a mixed hydrocarbons flow of the flow 4 and the flow 35;
k) under the process conditions of a temperature of 450 to 650°C, a reaction pressure of 0.01 to 2.0MPa and a weight space velocity of raw material(s) of 0.1 to 4 h⁻¹, contacting a hydrocarbons flow 33 with a catalyst in a second aromatization reactor to obtain a hydrocarbons flow 34, wherein said hydrocarbons flow 33 is selected from a mixed hydrocarbons flow of the flow 32 and a flow I, wherein the flow I is selected from a part or all of at least one of a C₂⁺ non-aromatic hydrocarbon flow 102 outside the reaction-separation system and a flow 39;
1) removing CO₂ and a part of acidic oxygenate to obtain a gas phase non-aromatic hydrocarbon flow 35, a liquid phase hydrocarbons flow 36 containing aromatic hydrocarbons and an aqueous phase from the hydrocarbons flow 34 through a separation unit 7;
m) further separating the flow 5 and the flow 36 according to one of the following four manners, and reacting:
   ml) for example, with reference to Figure 5, separating the flow 5 and the flow 36 in a separation unit 8 by a non-precise rectification to obtain a hydrocarbons flow 37 of aromatic hydrocarbons having less than or equal to 7 carbon numbers and a hydrocarbons flow 38 of aromatic hydrocarbons having more than 7 carbon numbers, and separating said flow 38 through a separation unit 9 to obtain the flow 39 containing non-aromatic hydrocarbons, a C₈ aromatic hydrocarbon flow 40 and a C₉⁺ aromatic hydrocarbon flow 41, and reacting said C₉⁺ aromatic hydrocarbon flow 41 or a mixed hydrocarbons flow of the C₉⁺ aromatic hydrocarbon flow 41 and optionally a C₉⁺ aromatic hydrocarbon flow 106 in a dealkylation reactor to obtain a Cg aromatic hydrocarbon flow 202;
      obtaining a hydrocarbons flow 2 of aromatic hydrocarbons having less than or equal to 7 carbon numbers from the hydrocarbons flow 37 and a flow H, wherein said flow H is selected from at least one of a C₂⁺ hydrocarbons flow 101 outside the reaction-separation system and the flow 39; and returning said hydrocarbons flow 2 to the oxygenate(s) flow 1 for further reaction;
   m2) for example, with reference to Figure 6, separating the flow 5 and the flow 36 in a separation unit 8 by a non-precise rectification to obtain a hydrocarbons flow 37 of aromatic hydrocarbons having less than or equal to 7 carbon numbers and a hydrocarbons flow 38 having more than 7 carbon numbers, and separating said flow 38 through a separation unit 9 to obtain the flow 39 containing non-aromatic hydrocarbons, a Cg - aromatic hydrocarbon flow 40 and a C₉⁺ aromatic hydrocarbon flow 41,
      obtaining a flow 44 containing C₈ aromatic hydrocarbons from a C₉⁺ aromatic hydrocarbon flow 42 and a methylbenzene flow 43 outside a reaction-separation system, wherein the C₉⁺ aromatic hydrocarbon flow 42 is selected from the flow 41 or a mixed flow of the flow 41 and a C₉⁺ aromatic hydrocarbon flow 106 outside the reaction-separation system,
      obtaining a hydrocarbons flow 2 of aromatic hydrocarbons having less than or equal to 7 carbon numbers from the hydrocarbons flow 37 and a flow H, wherein said flow H is selected from at least one of a C₂⁺ hydrocarbons flow 101 outside the reaction-separation system and the flow 39; and returning said hydrocarbons flow 2 to the oxygenate(s) flow 1 for further reaction;
   m3) for example, with reference to Figure 7, separating the flow 5 and the flow 36 in a separation unit 10 by a non-precise rectification to obtain a hydrocarbons flow 47 of aromatic hydrocarbons having less than or equal to 7 carbon numbers and a hydrocarbons flow 48 having more than 7 carbon numbers, and separating said flow 48 through a separation unit 11 to obtain the flow 49 containing non-aromatic hydrocarbons, a C₈ - aromatic hydrocarbon flow 50 and a C₉⁺ aromatic hydrocarbon flow 51;
      obtaining a hydrocarbons flow 2 of aromatic hydrocarbons having less than or equal to 7 carbon numbers from the flow 47 and a flow H, wherein said flow H is selected from at least one of C₂⁺ hydrocarbons flow 101 outside the reaction-separation system and the flow 49; and returning said hydrocarbons flow 2 to the oxygenate flow 1 for further reaction;
   m4) for example, with reference to Figure 8, separating the flow 5 and the flow 36 in a separation unit 9 by a non-precise rectification to obtain a hydrocarbons flow 47 of aromatic hydrocarbons having less than or equal to 7 carbon numbers and a hydrocarbons flow 48 having more than 7 carbon numbers, and separating said flow 48 through a separation unit 9 to obtain a flow 49 containing non-aromatic hydrocarbons, a Cg aromatic hydrocarbon flow 50 and a C₉⁺ aromatic hydrocarbon flow 51,
      obtaining a flow 54 containing Cg aromatic hydrocarbons from a C₉⁺ aromatic hydrocarbon flow 52 and a methylbenzene flow 53 outside the reaction-separation system, wherein the C₉⁺ aromatic hydrocarbon flow 52 is selected from the flow 51 or a mixed flow of the flow 51 and a C₉⁺ aromatic hydrocarbon flow 106 outside the reaction-separation system,
      obtaining a hydrocarbons flow 2 of aromatic hydrocarbons having less than or equal to 7 carbon numbers from the flow 47 and a flow H, wherein said flow H is selected from at least one of C₂⁺ hydrocarbon flow 101 outside the reaction-separation system or the flow 49; and returning said hydrocarbons flow 2 to the oxygenate flow 1 for further reaction.

The aromatization reactor used in the aromatization reaction may be at least one of a fluidized-bed reactor, a fixed-bed reactor and a moving-bed reactor. The aromatization reactor preferably is a fluidized-bed reactor and a moving-bed reactor, optionally with different regeneration systems or with a common regeneration system. The transalkylation reactor may be a fixed-bed reactor.

The aromatization catalyst comprise at least one molecular sieve active component selected from ZSM-11 and ZSM-5 molecular sieves, and the molar ratio of silicon oxide to aluminium oxide in the molecular sieve is 10:1 to 200:1. The transalkylation catalyst comprises at least one molecular sieve active component selected from MOR, ZSM-5 and BETA molecular sieves. The dealkylation process may be free of catalyst or has a catalyst selected from oxide and molecular sieve type dealkylation catalysts. The molecular sieve component prior to the molding of the aromatization catalyst or the catalyst supporting the modification component is preferably subjected to a high temperature hydro-thermal treatment, wherein the conditions of the hydro-thermal treatment are preferably a temperature of 400 to 750°C, a partial pressure of water vapor of 5 to 100%, a treatment time period of 0.5 to 120 hours, preferably 0.5 to 96 hours.

Molding manners such as band extrusion or tablet compressing are used in the fixed-bed catalyst. A molding manner of ball rolling may be used in the moving-bed catalyst. The fixed-bed catalyst and the moving-bed catalyst may contain a certain amount of adhesive components, such as amorphous silicon oxide, aluminium oxide or zirconium oxide and the like. In order to enhance the aromatization property of the fluidized-bed catalyst, for example the conversion capability of an oxygenate, the total yield of aromatic hydrocarbon, the selectivity of aromatic hydrocarbon and the hydrothermal stability of the catalyst, metal oxide or non-metal oxide for modification of catalyst may be introduced prior to or after molding of catalyst. Preferably a precursor component having a relatively good water solubility is used.

The fluidized-bed catalyst is produced by a spraying molding drying method. The fluidized-bed catalyst contains clay components for enhancing the strength and the antiwear property of fluidized-bed catalyst, such as kaolin, montmorillonite and hargil and the like. In addition, the components of the fluidized-bed catalyst further contain active components for adhering catalysts - molecular sieve and clay components, such as amorphous silicon oxide, aluminium oxide or zirconium oxide and the like. In order to enhance the aromatization property of the fluidized-bed catalyst, such as the conversion capability ofan oxygenate, the total yield of aromatic hydrocarbon, the selectivity of aromatic hydrocarbon and the hydrothermal stability of the catalyst, metal oxide or non-metal oxide for modification may be introduced to the composition of the catalyst.

In the process of the present invention, a mixed hydrocarbons flow of aromatic hydrocarbons having less than or equal to 7 carbon numbers enters the same aromatization reactor with an oxygenate. In the said aromatization reactor, in addition to the conversion of the oxygenate to an aromatic hydrocarbon, at least one of benzene or methylbenzene in the mixed hydrocarbons of aromatic hydrocarbons having less than or equal to 7 carbon numbers of the product can be converted to a dimethylbenzene product through alkylation reaction with the oxygenate, and thereby the added value of the aromatic hydrocarbon product is improved. Meanwhile, the non-aromatic hydrocarbon components in the mixed hydrocarbon flow of aromatic hydrocarbons having less than or equal to 7 carbon numbers of the product can be subjected to an aromatization reaction simultaneously and thus produce aromatic hydrocarbon to improve the total yield of aromatic hydrocarbon. In the reaction products, except a hydrocarbons flow M containing aromatic hydrocarbons having less than or equal to 7 carbon numbers, the non-aromatic hydrocarbon hydrocarbons components may enter at least one aromatization reactor for a cycle conversion to aromatic hydrocarbon so as to improve the yield of aromatic hydrocarbon during the process.

The aromatic hydrocarbon separation technique in the prior art separates the liquid phase product containing aromatic hydrocarbons having more than 5 carbon numbers by solvent extraction and rectification unit operation to obtain non-aromatic hydrocarbon components, benzene, methylbenzene, C₈ aromatic hydrocarbon and a heavy aromatic hydrocarbon having more than 9 carbon numbers one by one by a manner of precise separation. By doing so, both the energy consumption and material consumption during the separation process are relatively large, which will undoubtedly increase the cost of the production of aromatic hydrocarbon from the oxygenate greatly. As compared to the reaction-separation technique of aromatic hydrocarbon in the prior art, the solution used in the present invention firstly separates the liquid phase product containing aromatic hydrocarbons having more than 5 carbon numbers into a hydrocarbons flow of aromatic hydrocarbon having less than or equal to 7 carbon numbers and a hydrocarbons flow containing C₈ aromatic hydrocarbon by a non-precise separation manner. The hydrocarbons flow of aromatic hydrocarbon having less than or equal to 7 carbon numbers can enter the same aromatization reactor with the oxygenate without further separation. The benzene or methylbenzene component in the hydrocarbons flow of aromatic hydrocarbons having less than or equal to 7 carbon numbers can be subjected to an alkylation reaction with the oxygenate for a cycle conversion to dimethylbenzene. Meanwhile, the non-aromatic hydrocarbons component in the hydrocarbons flow containing Cg aromatic hydrocarbon may enter one or more aromatization reactor(s) for a cycle conversion to aromatic hydrocarbon to further improve the total yield of aromatic hydrocarbon. As compared to the prior art, using the reaction-separation solution provided in the present invention can partially or wholly omit the step of separating non-aromatic hydrocarbons from aromatic hydrocarbons and BTX aromatic hydrocarbons one by one and save a large amount of material consumption and energy consumption in separation and thus reduce the production cost during the process.

In the solution provided in the present invention, the removal of non-aromatized active H₂, CO, CO₂ and a part of acidic oxygenates from the gas phase non-aromatic hydrocarbon components through separation operation, followed by a cycle conversion in an aromatization reactor, prevents the accumulation of the above substances in reaction materials, improves the efficiency of the reactor and can effectively reduce a corrosion of acidic oxygenates on devices and pipelines. The present invention also provides a solution of incorporating transalkylation or dealkylation reactors. The C₉⁺ heavy aromatic hydrocarbons in the product are converted to dimethylbenzene by reacting with the methylbenzene in the product or outside the reaction-separation system in a dealkylation reactor, or the C₉⁺ heavy aromatic hydrocarbons in the product is converted to Cg aromatic hydrocarbon, such as dimethylbenzene, through dealkylation reaction in a dealkylation reactor, which improves the yield of Cg aromatic hydrocarbons and facilitates the improvement of the added value of the product.

To sum up, as compared to the prior art, the present invention comprises at least one aromatization reactor. A hydrocarbons mixture of aromatic hydrocarbons having less than or equal to 7 carbon numbers enters the same reactor directly with the oxygenate as raw material for reaction without complete precise separation. The residual non-aromatic hydrocarbons may enter one or more aromatization reactor(s) within the reaction-separation system. It can not only realize the aromatization of the oxygenate but also convert the non-aromatic hydrocarbons containing byproducts to aromatic hydrocarbon, and convert a hydrocarbons flow containing benzene and methylbenzene in the reaction product to aromatic hydrocarbon or a dimethylbenzene product having a high added value, which improves the total yield of aromatic hydrocarbon and the yield of dimethylbenzene of the above process. In addition, as compared to the prior art, in the solution provided in the present invention, the hydrocarbons mixture of aromatic hydrocarbons having less than or equal to 7 carbon numbers enters the same reactor directly with the oxygenate as raw material for reaction without precise separation, which greatly saves the energy consumption and the material consumption in separation.

### The description of Figures

Figure 1 is a flow chart of one embodiment of the present invention;
Figure 2 is a flow chart of one embodiment of the present invention;
Figure 3 is a flow chart of one embodiment of the present invention;
Figure 4 is a flow chart of one embodiment of the present invention;
Figure 5 is a flow chart of one embodiment of the present invention;
Figure 6 is a flow chart of one embodiment of the present invention;
Figure 7 is a flow chart of one embodiment of the present invention;
Figure 8 is a flow chart of one embodiment of the present invention;
Figure 9 is a flow chart of the separation manner of the flow Y of the present invention;
Figure 10 is a flow chart of the separation manner of the flow Y of the present invention.

### Specific embodiments

The present invention is further elaborated with the following specific Examples.

### Example 1

With reference to the reaction-separation flow chart of, for example, Figure 1, it had the following steps:
a) contacting the methanol flow 1 in an aromatization reactor with a catalyst for reaction under the process conditions of a temperature of 400°C, a pressure of 0.05MPa, a methanol weight space velocity of 0.1h⁻¹ to obtain the hydrocarbons flow 3;
b) removing CO₂ and a part of oxygenates from the flow 3 through the separation unit 1 to obtain the gas phase non-aromatic hydrocarbons flow 4, the liquid hydrocarbons flow 5 containing aromatic hydrocarbons and the aqueous phase;
c) removing inorganic gases such as H₂, CO, CO₂, N₂ and the like, CH₄ and a part of oxygenates from the flow 4 through the separation unit 2 via pressure swing adsorption to obtain a C₂⁺ hydrocarbons flow 6;
d) subjecting the hydrocarbons flow 5 to a non-precise rectification through the separation unit 3 to obtain the hydrocarbons flow 7 of aromatic hydrocarbons having less than or equal to 7 carbon numbers and a hydrocarbons flow 8 of aromatic hydrocarbons having more than 7 carbon numbers, and obtaining the hydrocarbons flow 9, the flow 10 containing Cg aromatic hydrocarbon and the C₉⁺ aromatic hydrocarbon flow 11 from the hydrocarbons flow 8 through the separation unit 4;
e) returning the above hydrocarbons flow 2 of aromatic hydrocarbons having less than or equal to 7 carbon numbers consisting of the C₂⁺ hydrocarbons flow 6, the hydrocarbons flow 7 and the hydrocarbons flow 9 to the above methanol flow 1 for further reaction;
f) reacting the C₉⁺ aromatic hydrocarbon flow 11 in a dealkylation reactor under the conditions of 750°C, a hydrocarbons weight space velocity of 8h⁻¹ and a reaction pressure of 5MPa to obtain a C₈ aromatic hydrocarbon flow 201, without any catalyst during this reaction process.

The aromatization reactor was of a fixed-bed form. The catalyst contained ZSM-5 molecular sieve active components having silicon oxide and aluminium oxide in a molar ratio of 10:1, wherein the catalyst was treated for 0.5 hour at 750°C and a water vapor partial pressure of 100% before use.

In the present Example, the conversion rate of methanol was more than 99%; the yield of dimethylbenzene was 81.5% and the total yield of aromatic hydrocarbon was 84.8%, based on the weight of carbon and hydrogen of methanol.

### Example 2

With reference to the reaction-separation flow chart of, for example, Figure 2, it had the following steps.
a) contacting the methanol flow 1 in an aromatization reactor with a catalyst for reaction under the process conditions of a temperature of 550°C, a pressure of 2.0MPa, a methanol weight space velocity of 4.0h⁻¹ to obtain the hydrocarbons flow 3;
b) removing CO₂ and a part of oxygenates from the flow 3 through the separation unit 1 to obtain the gas phase non-aromatic hydrocarbons flow 4, the liquid hydrocarbons flow 5 containing aromatic hydrocarbons and the aqueous phase;
c) removing inorganic gases such as H₂, CO, CO₂, N₂ and the like, CH₄ and a part of oxygenates from the flow 4 through the separation unit 2 via pressure swing adsorption to obtain the C₂⁺ hydrocarbons flow 6;
d) subjecting the hydrocarbons flow 5 to a non-precise rectification through the separation unit 3 to obtain the hydrocarbons flow 7 of aromatic hydrocarbons having less than or equal to 7 carbon numbers and the hydrocarbons flow 8 of aromatic hydrocarbons having more than 7 carbon numbers, and obtaining the hydrocarbons flow 9 containing non-aromatic hydrocarbon, the flow 10 containing C₈ aromatic hydrocarbon and the C₉⁺ aromatic hydrocarbon flow 11 from the hydrocarbons flow 8 through the separation unit 4;
e) returning the above hydrocarbons flow 2 of aromatic hydrocarbons having less than or equal to 7 carbon numbers consisting of the C₂⁺ hydrocarbons flow 6, the hydrocarbons flow 7 and the hydrocarbons flow 9 containing non-aromatic hydrocarbons and the C₂⁺ hydrocarbons flow 101 outside the reaction-separation system to the above methanol flow 1 for further reaction, wherein the hydrocarbons flow 101 had a weight composition of 43% ethylene, 32% propylene, 20%1-butene and 5% n-butane, and the C₂⁺ hydrocarbons flow 101 and the methanol flow 1 has a weight ratio of 1: 10;
f) forming the above C₉⁺ aromatic hydrocarbon flow 12 with the above C₉⁺ aromatic hydrocarbon flow 11, and contacting the C₉⁺ aromatic hydrocarbon flow 12, the methylbenzene flow 13 outside the reaction-separation system through a dealkylation reactor under the conditions of a temperature of 350°C, a hydrogen pressure of 5.0MPa and a weight space velocity of raw material(s) of 0.1h⁻¹ with a catalyst to obtain the hydrocarbons flow 15 containing dimethylbenzene.

The aromatization reactor was of a fluidized-bed form and the transalkylation reactor was of a fixed-bed reactor form. The catalyst contained ZSM-5 molecular sieve active components having silicon oxide and aluminium oxide in a molar ratio of 10:1, wherein the catalyst was treated for 0.5 hour at 750°C and a water vapor partial pressure of 100% before use.

In the present Example, the conversion rate of methanol was more than 99%; the yield of dimethylbenzene was 90.5% and the total yield of aromatic hydrocarbon was 98.9%, based on the weight of carbon and hydrogen of methanol.

### Example 3

With reference to the reaction-separation flow chart of, for example, Figure 3, it had the following steps:
a) contacting the dimethyl ether flow 1 in an aromatization reactor with a catalyst for reaction under the process conditions of a temperature of 480°C, a pressure of 0.3MPa, a weight space velocity of raw material of 1.5h⁻¹ to obtain the hydrocarbons flow 3;
b) removing CO₂ and a part of oxygenates from the flow 3 through the separation unit 1 to obtain the gas phase non-aromatic hydrocarbons flow 4, the liquid hydrocarbons flow 5 containing aromatic hydrocarbons and the aqueous phase;
c) removing inorganic gases such as H₂, CO, CO₂, N₂ and the like, CH₄ and a part of oxygenates from the flow 4 through the separation unit 2 via rectification to obtain the C₂⁺ hydrocarbons flow 6;
d) subjecting the hydrocarbons flow 5 to a non-precise rectification through the separation unit 5 to obtain the hydrocarbons flow 21 of aromatic hydrocarbons having less than or equal to 6 carbon numbers and a hydrocarbons flow 22 of aromatic hydrocarbons having more than 6 carbon numbers, and obtaining the hydrocarbons flow 23 containing non-aromatic hydrocarbons, the methylbenzene flow 24, the flow 25 containing Cg aromatic hydrocarbons and the C₉⁺ aromatic hydrocarbon flow 26 from the flow 22 through the separation unit 6;
e) returning the above hydrocarbon flow 2 of aromatic hydrocarbons having less than or equal to 7 carbon numbers consisting of the C₂⁺ hydrocarbons flow 6, the hydrocarbons flow 21 and the hydrocarbons flow 23 containing non-aromatic hydrocarbons to the above dimethyl ether flow 1 for further reaction.

The aromatization reactor was of a fluidized-bed reactor form. The catalyst contained ZSM-5 molecular sieve active components having silicon oxide and aluminium oxide in a molar ratio of 30: 1, wherein the catalyst was treated for 120 hours at 400°C and a water vapor partial pressure of 50% before use.

In the present Example, the conversion rate of dimethyl ether was more than 99%; the yield of dimethylbenzene was 82.5% and the total yield of aromatic hydrocarbon is 88.2%, based on the weight of carbon and hydrogen of dimethyl ether.

### Example 4

With reference to the reaction-separation flow chart of, for example, Figure 4, it had the following steps.
a) contacting the dimethyl ether flow 1 in an aromatization reactor with a catalyst for reaction under the process conditions of a temperature of 520°C, a pressure of 0.3MPa, a weight space velocity of raw material of 0.8h⁻¹ to obtain the hydrocarbons flow 3;
b) removing CO₂ and a part of oxygenates from the flow 3 through the separation unit 1 to obtain the gas phase non-aromatic hydrocarbons flow 4, the liquid hydrocarbons flow 5 containing aromatic hydrocarbons and the aqueous phase;
c) removing inorganic gases such as H₂, CO, CO₂, N₂ and the like, CH₄ and a part of oxygenates from the flow 4 through the separation unit 2 via rectification to obtain the C₂⁺ hydrocarbons flow 6;
d) subjecting the hydrocarbons flow 5 to a non-precise rectification through the separation unit 5 to obtain a hydrocarbons flow 21 of aromatic hydrocarbons having less than or equal to 6 carbon numbers and the hydrocarbons flow 22 of aromatic hydrocarbons having more than 6 carbon numbers, and obtaining the hydrocarbons flow 23 containing non-aromatic hydrocarbons, the methylbenzene flow 24, the flow 25 containing C₈ aromatic hydrocarbons and the C₉⁺ aromatic hydrocarbon flow 26 from the flow 22 through the separation unit 6;
e) returning the above hydrocarbons flow 2 of aromatic hydrocarbons having less than or equal to 7 carbon numbers consisting of 95% by weight of the flow 6, the hydrocarbons flow 21 and the hydrocarbons flow 23 containing non-aromatic hydrocarbons to the above dimethyl ether flow 1 for further reaction;
f) the methylbenzene flow 24 and the methylbenzene flow 105 outside the reaction-separation system formed the mixed flow 27, wherein the weight ratio of the methylbenzene flow 105 to the methylbenzene flow 24 was 20:80, and the C₉⁺ aromatic hydrocarbon flow 26 formed the C₉⁺ aromatic hydrocarbon flow 28; contacting the mixed flow 27 and the C₉⁺ aromatic hydrocarbon flow 28 in a transalkylation reactor under the reaction conditions of a temperature of 550°C, a hydrogen pressure of 0.5MPa and a weight space velocity of raw material of 10h⁻¹ with a catalyst to obtain the flow 29 containing dimethylbenzene.

The aromatization reactor was of a fluidized-bed reactor form. The catalyst contained ZSM-5 molecular sieve active components having silicon oxide and aluminium oxide in a molar ratio of 100:1, wherein the catalyst was treated for 60 hours at 550°C and a water vapor partial pressure of 75% before use.

In the present Example, the conversion rate of dimethyl ether was more than 99%; the yield of dimethylbenzene was 88.3% and the total yield of aromatic hydrocarbons was 94.8%, based on the weight of carbon and hydrogen of dimethyl ether.

### Example 5

With reference to the reaction-separation flow chart of, for example, Figure 5, it had the following steps:
h) contacting the methanol flow 1 in the first aromatization reactor with a catalyst for reaction under the process conditions of a temperature of 450°C, a pressure of 0.05MPa, a weight space velocity of raw material of 1.0h⁻¹ to obtain the hydrocarbons flow 3;
i) removing CO₂ and a part of acidic oxygenates from the flow 3 through the separation unit 1 to obtain the gas phase non-aromatic hydrocarbons flow 4, the liquid hydrocarbons flow 5 containing aromatic hydrocarbons and the aqueous phase;
j) removing inorganic gases such as H₂, CO, CO₂, N₂ and the like, CH₄ and a part of oxygenates from the hydrocarbons flow 31 through the separation unit 2 via C₅-C₉ gasoline absorption to obtain the C₂⁺ non-aromatic hydrocarbons flow 32, wherein the hydrocarbon flow 31 was a mixed hydrocarbon flow of the flow 4 and the gas phase non-aromatic hydrocarbon flow 35;
k)contacting the mixed hydrocarbons flow 33 in the second aromatization reactor under the process conditions of a temperature of 450°C, a pressure of 0.1MPa, a weight space velocity of raw material of 0.1h⁻¹ to obtain the hydrocarbons flow 34, wherein the hydrocarbons flow 33 was a mixed hydrocarbons flow of the flow 32 and the flow I which was selected from a part or all of at least one of the C₂⁺ non-aromatic hydrocarbons flow 102 outside the reaction-separation system and the flow 39;
1) removing CO₂ and a part of acidic oxygenates from the hydrocarbons flow 34 through the separation unit 7 to obtain the gas phase non-aromatic hydrocarbons flow 35, the liquid hydrocarbons flow 36 containing aromatic hydrocarbons and the aqueous phase;
m) separating the flow 5 and the flow 36 in the separation unit 8 through a non-precise rectification to obtain the hydrocarbon flow 37 of aromatic hydrocarbons having less than or equal to 7 carbon numbers and the hydrocarbons flow 38 of aromatic hydrocarbons having more than 7 carbon numbers, and separating the flow 38 through the separation unit 9 to obtain the flow 39 containing non-aromatic hydrocarbons, the Cg aromatic hydrocarbon flow 40 and the C₉⁺aromatic hydrocarbon flow 41;
n) returning the hydrocarbons flow 2 of aromatic hydrocarbons having less than or equal to 7 carbon numbers formed by the flow 37 and the flow 39 to the methanol flow 1 for further reaction;
o) contacting the C₉⁺aromatic hydrocarbon flow 41 in a fixed-bed dealkylation reactor under the reaction conditions of a reaction temperature of 350°C, a reaction pressure of 3MPa, a molar ratio of hydrogen to hydrocarbons of 10: 1 and a hydrocarbons weight space velocity of 4h⁻¹ with a Pt/ZSM-5 catalyst for reaction to obtain the Cg aromatic hydrocarbon flow 202;

The first aromatization reactor was of a fluidized-bed reactor form and the second aromatization reactor was of a fixed-bed reactor form; the aromatization catalyst contained ZSM-5 molecular sieve active components having silicon oxide and aluminium oxide in a molar ratio of 100: 1, wherein the catalyst was treated for 60 hours at 550°C and a water vapor partial pressure of 75% before use.

In the present Example, the conversion rate of methanol was more than 99.9%; the yield of dimethylbenzene was 82.0% and the total yield of aromatic hydrocarbon was 89.7%, based on the weight of carbon and hydrogen of methanol.

### Example 6

With reference to the reaction-separation flow chart of, for example, Figure 6, it had the following steps.
h) contacting the methanol flow 1 in the first aromatization reactor with a catalyst for reaction under the process conditions of a temperature of 550°C, a pressure of 2.0MPa, a weight space velocity of raw material of 4h⁻¹ to obtain the hydrocarbons flow 3;
i) removing CO₂ and a part of acidic oxygenates from the flow 3 through the separation unit 1 to obtain the gas phase non-aromatic hydrocarbons flow 4, the liquid hydrocarbons flow 5 containing aromatic hydrocarbons and the aqueous phase;
j) removing inorganic gases such as H₂, CO, CO₂, N₂ and the like, CH₄ and a part of oxygenates from the hydrocarbons flow 31 through the separation unit 2 via rectification to obtain the C₂⁺ non-aromatic hydrocarbons flow 32, wherein the hydrocarbons flow 31 was a mixed hydrocarbon flow of the flow 4 and 95% by weight of the flow 35;
k)contacting the hydrocarbons flow 33 in the second aromatization reactor under the process conditions of a temperature of 650°C, a pressure of 1.0MPa, a weight space velocity of raw material of 4h⁻¹ with a catalyst to obtain the hydrocarbons flow 34, wherein the hydrocarbons flow 33 was a mixed flow of the flow 32 and the C₂⁺ non-aromatic hydrocarbons flow 102 whose weight composition was 35% ethylene, 5%ethane, 29%propylene, 12% propane, 11% 1-butene, 7%n-butane, and the weight ratio of the C₂⁺ non-aromatic hydrocarbon flow 102 to the flow 32 was 0.5:1;
l)removing CO₂ and a part of acidic oxygenate to obtain the gas phase non-aromatic hydrocarbon flow 35, the liquid phase hydrocarbons flow 36 containing aromatic hydrocarbons and the aqueous phase from the hydrocarbons flow 34 through the separation unit 7;
m) separating the flow 5 and the flow 36 in the separation unit 8 through a non-precise rectification to obtain the hydrocarbons flow 37 of aromatic hydrocarbons having less than or equal to 7 carbon numbers and the hydrocarbons flow 38 having more than 7 carbon numbers, and separating the flow 38 through the separation unit 9 to obtain the flow 39 containing non-aromatic hydrocarbons, the C₈ aromatic hydrocarbon flow 40 and the C₉⁺aromatic hydrocarbon flow 41;
n) returning the hydrocarbons flow 2 of aromatic hydrocarbons having less than or equal to 7 carbon numbers formed by the flow 37 and the flow 39 to the methanol flow 1 for further reaction;
o) forming a C₉⁺aromatic hydrocarbon flow 42 by the flow 41, and contacting the C₉⁺aromatic hydrocarbon flow 42 and the methylbenzene 43 outside the reaction-separation system in the dealkylation reactor under a reaction temperature of 400°C, a reaction pressure of 3.0MPa, a weight space velocity of raw material of 4h⁻¹ with a catalyst for reaction to obtain a C₈ aromatic hydrocarbon flow 44;
   wherein, both the first aromatization reactor and the second aromatization reactor were of a fluidized-bed reactor form and the same catalyst were used, wherein the catalyst contained ZSM-11 molecular sieve active components having silicon oxide and aluminium oxide in a molar ratio of 75:1 and the catalyst was treated for 16 hours at 700°C and a water vapor partial pressure of 30% before use.

In the present Example, the conversion rate of methanol was more than 99.9%; the yield of dimethylbenzene was 91.7% and the total yield of aromatic hydrocarbon was 99.2%, based on the weight of carbon and hydrogen of methanol.

### Example 7

With reference to the reaction-separation flow chart of, for example, Figure 7, it had the following steps:
h) contacting an methanol flow 1 in the first aromatization reactor with a catalyst for reaction under the process conditions of a temperature of 500°C, a pressure of 0.5MPa, a weight space velocity of raw material of 0.8h⁻¹ to obtain the hydrocarbons flow 3;
i) removing CO₂ and a part of acidic oxygenates from the flow 3 through the separation unit 1 to obtain the gas phase non-aromatic hydrocarbons flow 4, the liquid hydrocarbons flow 5 containing aromatic hydrocarbons and the aqueous phase;
j) removing inorganic gases such as H₂, CO, CO₂, N₂ and the like, CH₄ and a part of oxygenates from the hydrocarbons flow 31 through the separation unit 2 via rectification to obtain the C₂⁺ non-aromatic hydrocarbon flow 32, wherein the hydrocarbons flow 31 was a mixed hydrocarbon flow of the flow 4 and the gas phase non-aromatic hydrocarbons flow 35;
k)contacting the hydrocarbons flow 33 in the second aromatization reactor under the process conditions of a temperature of 600°C, a pressure of 0.3MPa, a weight space velocity of raw material of 1.0h⁻¹ with a catalyst to obtain the hydrocarbons flow 34, wherein the hydrocarbons flow 33 was from a flow 32;
1) removing CO₂ and a part of acidic oxygenate(s) to obtain the gas phase non-aromatic hydrocarbon flow 35, the liquid phase hydrocarbon flow 36 containing aromatic hydrocarbons and the aqueous phase from the hydrocarbons flow 34 through the separation unit 7;
m) separating the flow 5 and the flow 36 in the separation unit 10 through a non-precise rectification to obtain the hydrocarbons flow 47 of aromatic hydrocarbons having less than or equal to 7 carbon numbers and the hydrocarbons flow 48 having more than 7 carbon numbers, and separating the flow 48 through the separation unit 11 to obtain the flow 49 containing non-aromatic hydrocarbons, the Cg aromatic hydrocarbon flow 50 and the C₉⁺aromatic hydrocarbon flow 51;
n) returning the hydrocarbons flow 2 of aromatic hydrocarbons having less than or equal to 7 carbon numbers formed by the flow 47 and the flow 49 to the methanol flow 1 for further reaction;
   wherein, both the first aromatization reactor and the second aromatization reactor were of a moving-bed reactor form and the same catalysts were used, wherein the catalyst contained ZSM-5 molecular sieve active components having silicon oxide and aluminium oxide in a molar ratio of 150:1 and the catalyst was treated for 24 hours at 600°C and a water vapor partial pressure of 60% before use.

In the present Example, the conversion rate of methanol was more than 99.9%; the yield of dimethylbenzene was 82.9% and the total yield of aromatic hydrocarbon was 88.5%, based on the weight of carbon and hydrogen of methanol.

### Example 8

With reference to the reaction-separation flow chart of, for example, Figure 8, it had the following steps.
h) contacting the methanol flow 1 and the hydrocarbons flow 2 of aromatic hydrocarbons having less than or equal to 7 carbon numbers in the first aromatization reactor with a catalyst for reaction under the process conditions of a temperature of 480°C, a pressure of 0.4MPa, a weight space velocity of raw material of 0.6h⁻¹ to obtain the hydrocarbons flow 3;
i) removing CO₂ and a part of acidic oxygenates from the flow 3 through the separation unit 1 to obtain the gas phase non-aromatic hydrocarbons flow 4, the liquid hydrocarbons flow 5 containing aromatic hydrocarbons and the aqueous phase;
j) removing inorganic gases such as H₂, CO, CO₂, N₂ and the like, CH₄ and a part of oxygenates from the hydrocarbons flow 31 through the separation unit 2 via pressure swing adsorption to obtain the C₂⁺ non-aromatic hydrocarbons flow 32, the hydrocarbons flow 31 is a mixed hydrocarbons flow of the flow 4 and the flow 35;
k) contacting the hydrocarbons flow 33 in the second aromatization reactor under the process conditions of a temperature of 610°C, a pressure of 0.3MPa, a weight space velocity of raw material of 0.8h⁻¹ with a catalyst to obtain the hydrocarbons flow 34, wherein the hydrocarbons flow 33 was the flow 32;
1) removing CO₂ and a part of acidic oxygenates to obtain the gas phase non-aromatic hydrocarbon flow 35, the liquid phase hydrocarbons flow 36 containing aromatic hydrocarbons and the aqueous phase from the hydrocarbons flow 34 through the separation unit 7;
m) separating the flow 5 and the flow 36 in the separation unit 9 through a non-precise rectification to obtain the hydrocarbons flow 47 of aromatic hydrocarbon having less than or equal to 7 carbon numbers and the hydrocarbons flow 48 having more than 7 carbon numbers, and separating the flow 48 through the separation unit 10 to obtain the flow 49 containing non-aromatic hydrocarbons, the C₈ aromatic hydrocarbon flow 50 and the C₉⁺aromatic hydrocarbon flow 51;
n) returning the hydrocarbons flow 2 of aromatic hydrocarbons having less than or equal to 7 carbon numbers formed by the flow 47 and the flow 49 to the methanol flow 1 for further reaction;
o) the mixed C₉⁺ aromatic hydrocarbon flow 52, which was formed by the flow 51 and the C₉⁺ aromatic hydrocarbon flow 106 outside the reaction-separation system, reacted with the methylbenzene flow 53 outside the reaction-separation system through a transalkylation reactor under a reaction temperature of 450°C, a hydrogen pressure of 4.0MPa, a weight space velocity of raw material of 2h⁻¹ to obtain the Cg aromatic hydrocarbon flow 54, wherein the weight ratio of the methylbenzene flow 53 outside the reaction-separation system to the C₉⁺ aromatic hydrocarbon flow 52 was 1: 5 and the weight ratio of the flow 106 to the flow 51 was 1:5;
   wherein, both the first aromatization reactor and the second aromatization reactor were of a fluidized-bed reactor form, the transalkylation reactor was of a fixed-bed form, and they comprised the same catalysts, wherein the catalyst contained ZSM-5 molecular sieve active components having silicon oxide and aluminium oxide in a molar ratio of 150: 1 and the catalyst was treated for 24 hours at 600°C and a water vapor partial pressure of 60% before use.

In the present Example, the conversion rate of methanol was more than 99.9%; the yield of dimethylbenzene was 87.6% and the total yield of aromatic hydrocarbon was 95.2%, based on the weight of carbon and hydrogen of methanol.

### Comparative Example 1

The reaction-separation procedure of producing aromatic hydrocarbons with an oxygenate comprised one aromatization reactor. The byproducts, i.e. non-aromatic hydrocarbons and benzene and methylbenzene, were taken as product directly, and were not subjected to a cycle conversion to aromatic hydrocarbons; the raw materials, the forms of aromatization reactors and the reaction conditions were the same as those of Example 1. The conversion rate of methanol was more than 99.9%, the total yield of aromatic hydrocarbon was 49.6% and the yield of dimethylbenzene was 29.8%.

### Comparative Example 2

The reaction-separation procedure of producing aromatic hydrocarbons with an oxygenate comprised one aromatization reactor; only benzene and methylbenzene in the product were returned to the reactor and converted to dimethylbenzene through alkylation. The raw materials, the forms of aromatization reactors, the reaction conditions and the operation conditions were the same as those of Example 3. The conversion rate of methanol was more than 99.9%, the total yield of aromatic hydrocarbon was 53.4% and the yield of dimethylbenzene was 35.2%.

## Claims

1. A method for producing an aromatic hydrocarbon with an oxygenate as raw material, comprising
i) reacting an oxygenate in at least one aromatization reactor to obtain an aromatization reaction product;
ii) separating the aromatization reaction product through a separation unit A to obtain a gas phase hydrocarbons flow X and a liquid phase hydrocarbons flow Y;
iii) after removing gas and/or a part of the oxygenate from the gas phase hydrocarbons flow X through a separation unit B, a reaction is conducted in another aromatization reactor and a separation is conducted through a separation unit A, to obtain a flow X2 containing a non-aromatic hydrocarbon and a flow X3 containing an aromatic hydrocarbon;
iv) a mixed hydrocarbons flow M of an aromatic hydrocarbon having less than or equal to 7 carbon numbers and a flow N of the residual hydrocarbons is obtained by a non-precise rectification of the liquid phase hydrocarbons flow Y in a separation unit C, optionally after combining said flow Y with the flow X3;
v) separating the flow N of the residual hydrocarbons through a separation unit D, to obtain a flow K containing a non-aromatic hydrocarbon, a C₈ aromatic hydrocarbon flow J and a C₉⁺ aromatic hydrocarbon flow L;
vi) returning
a) the flow X2 containing a non-aromatic hydrocarbon, and
b) the mixed hydrocarbons flow M of an aromatic hydrocarbon having less than or equal to 7 carbon numbers and/or a part or all of the flow K containing a non-aromatic hydrocarbon, and optionally
c) an additional C₂⁺ hydrocarbons flow,
to the above oxygenate; or
returning
a) the flow X2 containing a non-aromatic hydrocarbon, and
b) a mixed hydrocarbons flow M of an aromatic hydrocarbon having less than or equal to 7 carbon numbers and/or a part or all of the flow K containing a non-aromatic hydrocarbon,
to the aromatization reactor in iii);
vii) optionally, reacting the C₉⁺ aromatic hydrocarbons flow L in at least one reactor selected from a transalkylation reactor and a dealkylation reactor to obtain a C₈ aromatic hydrocarbon flow L1.

2. The method for producing an aromatic hydrocarbon with an oxygenate as raw material according to claim 1, wherein step iii) comprises a pressure swing adsorption, a rectification and/or an adsorption in the separation unit B and comprises operation units selected from quenching, alkaline washing and/or water washing in the separation unit A, and
wherein step v) comprises at least one of rectification and solvent extraction rectification in separation unit D.

3. The method for producing an aromatic hydrocarbon with an oxygenate as raw material according to claim 1, wherein the liquid phase hydrocarbons flow Y is separated by the following two manners:
1) flow Y enters a separation unit C1 and is separated by a non-precise rectification to obtain a mixed hydrocarbons flow M1 of aromatic hydrocarbons having less than or equal to 6 carbon numbers and a hydrocarbons flow N1 having more than 6 carbon numbers, and the hydrocarbons flow N1 enters a separation unit D1 to be able to obtain a C₈ aromatic hydrocarbon flow and a C₉⁺ aromatic hydrocarbon flow;
2) flow Y enters a separation unit C2 and is separated by a non-precise rectification to obtain a mixed hydrocarbons flow M2 of aromatic hydrocarbons having less than or equal to 7 carbon numbers and a hydrocarbons flow N2 having more than 7 carbon numbers, and the hydrocarbons flow N2 enters a separation unit D2 to be able to obtain a C₈ aromatic hydrocarbon flow and a C₉⁺ aromatic hydrocarbon flow.

4. The method for producing an aromatic hydrocarbon with an oxygenate as raw material according to any one of the claims 1 to 3, wherein a part or all of the non-aromatic hydrocarbon flow and the flow of the oxygenate come into contact with a catalyst for reaction in the same aromatization reactor or by entering different aromatization reactors; at least one reactor selected from the group consisting of a transalkylation reactor and a dealkylation reactor in the said method is used for converting a C₉⁺ aromatic hydrocarbon flow in the product of aromatic hydrocarbons to dimethylbenzene; the reaction conditions for said transalkylation reactor are a temperature of 350 to 550°C, a reaction pressure of 0.1 to 5.0MPa, a molar ratio of hydrogen/hydrocarbon of 1: 1 to 200: 1, a weight space velocity of raw material of 0.1 to 15 h⁻¹; the reaction conditions of said dealkylation reactor are a reaction temperature of 300 to 800°C, a molar ratio of hydrogen/hydrocarbon of 0.1 to 200: 1 and a weight space velocity of the hydrocarbons of 0.5 to 10 h⁻¹.

5. The method for producing an aromatic hydrocarbon with an oxygenate as raw material according to any one of claims 1 to 4, comprising one aromatization reactor, said method comprising the following steps:
a) under the process conditions of a temperature of 400 to 550°C, a pressure of 0.01 to 2.0MPa and a weight space velocity of raw material(s) of 0.1 to 4 h⁻¹, contacting a flow 1 of oxygenate(s) with a catalyst for reaction in an aromatization reactor to obtain a hydrocarbons flow 3;
b) removing CO₂ and a part of oxygenate(s) from said hydrocarbons flow 3 through a separation unit 1 to obtain a gas phase non-aromatic hydrocarbons flow 4, a liquid phase hydrocarbons flow 5 containing an aromatic hydrocarbon and an aqueous phase;
c) removing gas, CH₄ and a part of oxygenate(s) from said gas phase non-aromatic hydrocarbons flow 4 through a separation unit 2 to obtain a C₂⁺ hydrocarbons flow 6;
d) further separating the liquid phase hydrocarbons flow 5 containing an aromatic hydrocarbon according to one of the following four manners, and reacting:
d1) subjecting the liquid phase hydrocarbons flow 5 containing an aromatic hydrocarbon to non-precise rectification through a separation unit 3 to obtain a hydrocarbon flow 7 of aromatic hydrocarbons having less than or equal to 7 carbon numbers and a hydrocarbon flow 8 of aromatic hydrocarbons having more than 7 carbon numbers, and separating said hydrocarbons flow 8 through a separation unit 4 to obtain a hydrocarbons flow 9, a flow 10 containing C₈ aromatic hydrocarbon and a C₉⁺ aromatic hydrocarbon flow 11, and reacting said flow 11 in a dealkylation reactor to obtain a C₈ aromatic hydrocarbon flow 201;
obtaining a hydrocarbons flow 2 of aromatic hydrocarbons having less than or equal to 7 carbon numbers from the hydrocarbons flow 7 and a part or all of the C₂⁺ hydrocarbons flow 6, wherein said hydrocarbons flow 2 further optionally comprises a part or all of at least one selected from the hydrocarbons flow 9 and a C₂⁺ hydrocarbons flow 101 outside the reaction-separation system; and returning said hydrocarbons flow 2 to the oxygenate(s) flow 1 for further reaction;
d2) subjecting the liquid phase hydrocarbons flow 5 containing an aromatic hydrocarbon to a non-precise rectification through a separation unit 3 to obtain a hydrocarbon flow 7 of aromatic hydrocarbons having less than or equal to 7 carbon numbers and a hydrocarbons flow 8 of aromatic hydrocarbons having more than 7 carbon numbers, and obtaining a hydrocarbons flow 9 containing non-aromatic hydrocarbons, a flow 10 containing C₈ aromatic hydrocarbons and a C₉⁺ aromatic hydrocarbon flow 11 from said hydrocarbons flow 8 through a separation unit 4;
obtaining a hydrocarbon flow 15 containing dimethylbenzene from a C₉⁺ aromatic hydrocarbon flow 12 and a methylbenzene flow 13 outside the reaction-separation system through a transalkylation reactor, wherein the C₉⁺ aromatic hydrocarbon flow 12 is selected from one of a part or all of a C₉⁺ aromatic hydrocarbon flow 11 or a mixture of a part or all of the C₉⁺ aromatic hydrocarbon flow 11 and a C₉⁺ aromatic hydrocarbon flow 106 outside the reaction-separation system;
obtaining a hydrocarbons flow 2 of aromatic hydrocarbon having less than or equal to 7 carbon numbers from the hydrocarbons flow 7 and a part or all of a C₂⁺ hydrocarbons flow 6, wherein said hydrocarbons flow 2 further optionally comprises a part or all of at least one selected from the hydrocarbons flow 9 and a C₂⁺ hydrocarbons flow 101 outside the reaction-separation system; and returning said hydrocarbons flow 2 to the oxygenate(s) flow 1 for further reaction;
d3) subjecting the liquid phase hydrocarbons flow 5 containing an aromatic hydrocarbon to a non-precise rectification through a separation unit 5 to obtain a hydrocarbon flow 21 of aromatic hydrocarbon having less than or equal to 6 carbon numbers and a hydrocarbons flow 22 of aromatic hydrocarbon having more than 6 carbon numbers, and obtaining a hydrocarbons flow 23 containing non-aromatic hydrocarbons, a methylbenzene flow 24, a flow 25 containing C₈ aromatic hydrocarbons and a C₉⁺ aromatic hydrocarbon flow 26 from said flow 22 through a separation unit 6;
obtaining a hydrocarbons flow 2 of aromatic hydrocarbons having less than or equal to 7 carbon numbers from a part or all of the hydrocarbons flow 6 and the hydrocarbons flow 21, wherein said hydrocarbons flow 2 further optionally comprises a part or all of at least one selected from the hydrocarbons flow 23 and a C₂⁺ hydrocarbons flow 101 outside the reaction-separation system; and returning said hydrocarbons flow 2 to the oxygenate flow 1 for further reaction;
d4) subjecting the liquid phase hydrocarbons flow 5 containing an aromatic hydrocarbon to a non-precise rectification through a separation unit 5 to obtain a hydrocarbons flow 21 of aromatic hydrocarbons having less than or equal to 6 carbon numbers and a hydrocarbons flow 22 of aromatic hydrocarbons having more than 6 carbon numbers, and obtaining a hydrocarbon flow 23 containing non-aromatic hydrocarbons, a methylbenzene flow 24, a flow 25 containing C₈ aromatic hydrocarbons and a C₉⁺ aromatic hydrocarbon flow 26 from said flow 22 through a separation unit 6;
contacting a methylbenzene flow 27 and a C₉⁺ aromatic hydrocarbon flow 28 with a catalyst in a transalkylation reactor to obtain a flow 29 containing dimethylbenzene, wherein said flow 27 is selected from one of a part or all of the methylbenzene flow 24 or a mixed flow of a part or all of the flow 24 and a methylbenzene flow 105 outside the reaction-separation system, and said C₉⁺ aromatic hydrocarbon flow 28 is selected from one of a part or all of the flow 26 or a mixed flow of a part or all of the flow 26 and a C₉⁺ aromatic hydrocarbon flow 106 outside the reaction-separation system;
obtaining a hydrocarbons flow 2 of aromatic hydrocarbons having less than or equal to 7 carbon numbers from a part or all of the hydrocarbon flow 6 and the hydrocarbons flow 21, wherein said hydrocarbons flow 2 further optionally comprises a part or all of at least one selected from the flow 23 and a C₂⁺ hydrocarbons flow 101 outside the reaction-separation system; and returning said hydrocarbons flow 2 to the oxygenate(s) flow 1 for further reaction.

6. The method for producing an aromatic hydrocarbon with an oxygenate as raw material according to any one of claims 1 to 4, comprising two aromatization reactors, said method comprising the following steps:
h) under the process conditions of a temperature of 400 to 550°C, a reaction pressure of 0.01 to 2.0MPa and a weight space velocity of raw material(s) of 0.1 to 4 h⁻¹, contacting a flow 1 of oxygenate(s) with a catalyst for reaction in a first aromatization reactor to obtain a hydrocarbons flow 3;
i) removing CO₂ and a part of acidic oxygenate to obtain a gas phase non-aromatic hydrocarbon flow 4, a liquid phase hydrocarbons flow 5 containing aromatic hydrocarbons and an aqueous phase from a flow 3 through a separation unit 1;
j) removing gas, CH₄ and a part of oxygenate(s) from said hydrocarbons flow 31 through a separation unit 2 to obtain a C₂⁺ non-aromatic hydrocarbons flow 32, wherein the hydrocarbons flow 31 is a mixed hydrocarbons flow of the flow 4 and the flow 35;
k) under the process conditions of a temperature of 450 to 650°C, a reaction pressure of 0.01 to 2.0MPa and a weight space velocity of raw material(s) of 0.1 to 4 h⁻¹, contacting a hydrocarbons flow 33 with a catalyst in a second aromatization reactor to obtain a hydrocarbons flow 34, wherein said hydrocarbons flow 33 is selected from a mixed hydrocarbons flow of the flow 32 and a flow I, wherein the flow I is selected from a part or all of at least one of a C₂⁺ non-aromatic hydrocarbon flow 102 outside the reaction-separation system and a flow 39;
1) removing CO₂ and a part of acidic oxygenate to obtain a gas phase non-aromatic hydrocarbon flow 35, a liquid phase hydrocarbons flow 36 containing aromatic hydrocarbons and an aqueous phase from the hydrocarbons flow 34 through a separation unit 7;
m) further separating the flow 5 and the flow 36 according to one of the following four manners, and reacting:
m1) separating the flow 5 and the flow 36 in a separation unit 8 by a non-precise rectification to obtain a hydrocarbons flow 37 of aromatic hydrocarbons having less than or equal to 7 carbon numbers and a hydrocarbons flow 38 of aromatic hydrocarbons having more than 7 carbon numbers, and separating said flow 38 through a separation unit 9 to obtain the flow 39 containing non-aromatic hydrocarbons, a C₈ aromatic hydrocarbon flow 40 and a C₉⁺ aromatic hydrocarbon flow 41, and reacting said C₉⁺ aromatic hydrocarbon flow 41 or a mixed hydrocarbons flow of the C₉⁺ aromatic hydrocarbon flow 41 and optionally a C₉⁺ aromatic hydrocarbon flow 106 in a dealkylation reactor to obtain a C₈ aromatic hydrocarbon flow 202;
obtaining a hydrocarbons flow 2 of aromatic hydrocarbons having less than or equal to 7 carbon numbers from the hydrocarbons flow 37 and a flow H, wherein said flow H is selected from at least one of a C₂⁺ hydrocarbons flow 101 outside the reaction-separation system and the flow 39; and returning said hydrocarbons flow 2 to the oxygenate(s) flow 1 for further reaction;
m2) separating the flow 5 and the flow 36 in a separation unit 8 by a non-precise rectification to obtain a hydrocarbons flow 37 of aromatic hydrocarbons having less than or equal to 7 carbon numbers and a hydrocarbons flow 38 having more than 7 carbon numbers, and separating said flow 38 through a separation unit 9 to obtain the flow 39 containing non-aromatic hydrocarbons, a C₈ aromatic hydrocarbon flow 40 and a C₉⁺ aromatic hydrocarbon flow 41,
obtaining a flow 44 containing C₈ aromatic hydrocarbons from a C₉⁺ aromatic hydrocarbon flow 42 and a methylbenzene flow 43 outside a reaction-separation system, wherein the C₉⁺ aromatic hydrocarbon flow 42 is selected from the flow 41 or a mixed flow of the flow 41 and a C₉⁺ aromatic hydrocarbon flow 106 outside the reaction-separation system,
obtaining a hydrocarbons flow 2 of aromatic hydrocarbons having less than or equal to 7 carbon numbers from the hydrocarbons flow 37 and a flow H, wherein said flow H is selected from at least one of a C₂⁺ hydrocarbons flow 101 outside the reaction-separation system and the flow 39; and returning said hydrocarbons flow 2 to the oxygenate(s) flow 1 for further reaction;
m3) separating the flow 5 and the flow 36 in a separation unit 10 by a non-precise rectification to obtain a hydrocarbons flow 47 of aromatic hydrocarbons having less than or equal to 7 carbon numbers and a hydrocarbons flow 48 having more than 7 carbon numbers, and separating said flow 48 through a separation unit 11 to obtain the flow 49 containing non-aromatic hydrocarbons, a C₈ aromatic hydrocarbon flow 50 and a C₉⁺ aromatic hydrocarbon flow 51;
obtaining a hydrocarbons flow 2 of aromatic hydrocarbons having less than or equal to 7 carbon numbers from the flow 47 and a flow H, wherein said flow H is selected from at least one of C₂⁺ hydrocarbons flow 101 outside the reaction-separation system and the flow 49; and returning said hydrocarbons flow 2 to the oxygenate flow 1 for further reaction;
m4) separating the flow 5 and the flow 36 in a separation unit 9 by a non-precise rectification to obtain a hydrocarbons flow 47 of aromatic hydrocarbons having less than or equal to 7 carbon numbers and a hydrocarbons flow 48 having more than 7 carbon numbers, and separating said flow 48 through a separation unit 9 to obtain a flow 49 containing non-aromatic hydrocarbons, a C₈ aromatic hydrocarbon flow 50 and a C₉⁺ aromatic hydrocarbon flow 51,
obtaining a flow 54 containing C₈ aromatic hydrocarbons from a C₉⁺ aromatic hydrocarbon flow 52 and a methylbenzene flow 53 outside the reaction-separation system, wherein the C₉⁺ aromatic hydrocarbon flow 52 is selected from the flow 51 or a mixed flow of the flow 51 and a C₉⁺ aromatic hydrocarbon flow 106 outside the reaction-separation system,
obtaining a hydrocarbons flow 2 of aromatic hydrocarbons having less than or equal to 7 carbon numbers from the flow 47 and a flow H, wherein said flow H is selected from at least one of C₂⁺ hydrocarbon flow 101 outside the reaction-separation system or the flow 49; and returning said hydrocarbons flow 2 to the oxygenate flow 1 for further reaction.

7. The method for producing an aromatic hydrocarbon with an oxygenate as raw material according to any one of claims 1 to 6, **characterized in that** said aromatization reactor may be at least one of a fluidized-bed reactor, a fixed-bed reactor or a moving-bed reactor, and said transalkylation reactor and dealkylation reactor are fixed-bed reactors.

8. The method for producing an aromatic hydrocarbon with an oxygenate as raw material according to any one of claims 1 to 7, **characterized in that** said aromatization reactor is a fluidized-bed reactor and a moving-bed reactor, optionally with different regeneration systems or with a common regeneration system.

9. The method for producing an aromatic hydrocarbon with an oxygenate as raw material according to any one of claims 1 to 8, **characterized in that** said aromatization catalyst comprises at least one molecular sieve active component selected from ZSM-5 and ZSM-11 molecular sieves, wherein the molar ratio of silicon oxide to aluminium oxide in the molecular sieve is from 10: 1 to 200: 1.

10. The method for producing an aromatic hydrocarbon with an oxygenate as raw material according to any one of claims 1 to 8, **characterized in that** said transalkylation catalyst comprises at least one molecular sieve active component selected from MOR, ZSM-5 and BETA molecular sieves, and said dealkylation process may be free of catalyst or comprises oxide, molecular sieve type dealkylation catalyst.

11. The method for producing an aromatic hydrocarbon with an oxygenate as raw material according to any one of claims 1 to 10, **characterized in that** the molecular sieve component prior to the molding of the catalyst or the catalyst supporting the modification component is subjected to a high temperature hydro-thermal treatment under the conditions of a temperature of 400 to 750°C, a partial pressure of water vapor of 5 to 100%, a treatment time period of 0.5 to 120 hours.

## Patentansprüche

1. Verfahren zur Erzeugung eines aromatischen Kohlenwasserstoffes mit einem Oxygenat als Ausgangsmaterial, enthaltend
i) Reaktion eines Oxygenates in zumindest einem Aromatisierungsreaktor, unter Erhalt eines Aromatisierungs-Reaktionsproduktes,
ii) Trennen des Aromatisierungs-Reaktionsproduktes durch eine Trenneinheit A, unter Erhalt eines Gasphasen-Kohlenwasserstoffflusses X und eines Flüssigphasen-Kohlenwasserstoffflusses Y,
iii) nach Entfernen von Gas und/oder einem Teil des Oxygenates von dem Gasphasen-Kohlenwasserstofffluss X durch eine Trenneinheit B, Durchführen einer Reaktion in einem anderen Aromatisierungsreaktor und Durchführen einer Trennung durch eine Trenneinheit A, unter Erhalt eines Flusses X2, der einen nicht-aromatischen Kohlenwasserstoff enthält, und eines Flusses X3, der einen aromatischen Kohlenwasserstoff enthält,
iv) ein gemischter Kohlenwasserstofffluss M aus einem aromatischen Kohlenwasserstoff mit gleich oder weniger als 7 Kohlenstoffatomen und einem Fluss N aus den restlichen Kohlenwasserstoffen wird durch eine nicht genaue Rektifizierung des Flüssigphasen-Kohlenwasserstoffflusses Y in einer Trenneinheit C durchgeführt, wahlweise nach Kombinieren des Flusses Y mit dem Fluss X3,
v) Trennen des Flusses N der restlichen Kohlenwasserstoffe durch eine Trenneinheit D, unter Erhalt eines Flusses K, der einen nicht-aromatischen Kohlenwasserstoff enthält, eines C₈-aromatischen Kohlenwasserstoffflusses J und eines C₉⁺-aromatischen Kohlenwasserstoffflusses L,
vi) Zurückführen von
a) dem Fluss X2, der einen nicht-aromatischen Kohlenwasserstoff enthält, und
b) des gemischten Kohlenwasserstoffflusses M aus einem aromatischen Kohlenwasserstoff mit gleich oder weniger als 7 Kohlenstoffatomen und/oder eines Teils oder des gesamten Flusses K, der einen nicht-aromatischen Kohlenwasserstoff enthält, und wahlweise
c) einen zusätzlichen C₂⁺-Kohlenwasserstofffluss, zu dem obigen Oxygenat oder
Zurückführen
a) des Flusses X2, der einen nicht-aromatischen Kohlenwasserstoff enthält, und
b) eines gemischten Kohlenwasserstoffflusses M aus einem aromatischen Kohlenwasserstoff mit gleich oder weniger als 7 Kohlenstoffatomen und/oder eines Teils oder des Gesamten des Flusses K, der einen nicht-aromatischen Kohlenwasserstoff enthält,
zu dem Aromatisierungsreaktor in iii),
vii) wahlweises Reagieren des C₉⁺-aromatischen Kohlenwasserstoffflusses L in zumindest einem Reaktor, ausgewählt aus einem Transalkylierungsreaktor und einem Dealkylierungsreaktor, unter Erhalt eines C₈-aromatischen Kohlenwasserstoffflusses L1.

2. Verfahren zur Erzeugung eines aromatischen Kohlenwasserstoffs mit einem Oxygenat als Ausgangsmaterial gemäß Anspruch 1, worin Schritt iii) eine Druckwechseladsorption, eine Rektifizierung und/oder eine Adsorption in der Trenneinheit W enthält und Betriebseinheiten enthält, ausgewählt aus Abschrecken, alkalischem Waschen und/oder Waschen mit Wasser in der Trenneinheit A und
worin Schritt v) zumindest eines von der Rektifizierung und Lösungsmittel-Extraktions-Rektifizierung in der Trenneinheit D enthält.

3. Verfahren zur Erzeugung eines aromatischen Kohlenwasserstoffs mit einem Oxygenat als Ausgangsmaterial gemäß Anspruch 1, worin der Flüssigphasen-Kohlenwasserstofffluss Y durch die folgenden beiden Arten getrennt wird:
1) der Fluss Y betritt die Trenneinheit C1 und wird durch eine nicht genaue Rektifizierung getrennt, unter Erhalt eines gemischten Kohlenwasserstoffflusses M1 aus aromatischen Kohlenwasserstoffen mit gleich oder weniger als 6 Kohlenstoffatomen und eines Kohlenwasserstoffflusses M1 mit mehr als 6 Kohlenstoffatomen, und der Kohlenwasserstofffluss N1 betritt eine Trenneinheit D1, so dass ein aromatischer C₈-aromatischer Kohlenwasserstofffluss und ein aromatischer C₉⁺ Kohlenwasserstofffluss erhalten werden kann,
2) der Fluss Y betritt eine Trenneinheit C2 und wird durch eine nicht-präzise Rektifizierung getrennt, unter Erhalt eines gemischten Kohlenwasserstoffflusses M2 aus aromatischen Kohlenwasserstoffen mit gleich oder weniger als 7 Kohlenstoffatomen und eines Kohlenwasserstoffflusses N2 mit mehr als 7 Kohlenstoffatomen, und der Kohlenwasserstofffluss N2 betritt eine Trenneinheit D2, so dass ein aromatischer C₈-Kohlenwasserstofffluss und ein C₉⁺-Kohlenwasserstofffluss erhalten werden können.

4. Verfahren zur Erzeugung eines aromatischen Kohlenwasserstoffs mit einem Oxygenat als Ausgangsmaterial gemäß einem der Ansprüche 1 bis 3, worin ein Teil oder das Gesamte des nicht-aromatischen Kohlenwasserstoffflusses und der Fluss aus dem Oxygenat mit einem Katalysator für die Reaktion in dem gleichen Aromatisierungsreaktor oder durch Eintreten in verschiedene Aromatisierungsreaktoren kontaktieren können, wobei zumindest ein Reaktor, ausgewählt aus der Gruppe, bestehend aus einem Transalkylierungsreaktor und einem Dealkylierungsreaktor in dem Verfahren, zum Umwandeln eines aromatischen C₉⁺-Kohlenwasserstoffflusses in dem Produkt aus aromatischen Kohlenwasserstoffen in Dimethylbenzol verwendet wird; die Reaktionsbedingungen für den Transalkylierungsreaktor eine Temperatur von 350 bis 550°C, ein Reaktionsdruck von 0,1 bis 5,0 MPa, ein molares Verhältnis von Wasserstoff/Kohlenwasserstoff von 1:1 bis 200:1, eine Gewicht-Raumgeschwindigkeit des Ausgangsmaterials von 0,1 bis 15 h⁻¹ sind, die Reaktionsbedingungen des Dealkylierungsreaktors eine Reaktionstemperatur von 300 bis 800°C, ein molares Verhältnis von Wasserstoff/Kohlenwasserstoff von 0,1 bis 200:1 und eine Gewicht-Raumgeschwindigkeit der Kohlenwasserstoffe von 0,5 bis 10 h⁻¹ sind.

5. Verfahren zur Erzeugung eines aromatischen Kohlenwasserstoffs mit einem Oxygenat als Ausgangsmaterial gemäß einem der Ansprüche 1 bis 4, enthaltend einen Aromatisierungsreaktor, wobei das Verfahren die folgenden Schritte enthält:
a) unter den Prozessbedingungen einer Temperatur von 400 bis 550°C, eines Drucks von 0,01 bis 2,0 MPa und einer Gewicht-Raumgeschwindigkeit der Ausgangsmaterialien von 0,1 bis 4 h⁻¹ wird ein Fluss 1 aus Oxygenat(en) mit einem Katalysator für eine Reaktion in einem Aromatisierungsreaktor kontaktiert, unter Erhalt eines Kohlenwasserstoffflusses 3,
b) Entfernen von CO₂ und eines Teils von Oxygenat(en) von dem Kohlenwasserstofffluss 3 durch eine Trenneinheit 1, unter Erhalt eines Gasphasen-nicht-aromatischen Kohlenwasserstoffflusses 4, eines Flüssigphasen-Kohlenwasserstoffflusses 5, der einen aromatischen Kohlenwasserstoff enthält, und einer wässrigen Phase,
c) Entfernen von Gas, CH₄, und eines Teils von Oxygenat(en) von dem Gasphasen-nicht-aromatischen Kohlenwasserstofffluss 4 durch eine Trenneinheit 2, unter Erhalt eines C₂⁺-Kohlenwasserstoffflusses 6,
d) weiteres Trennen des Flüssigphasen-Kohlenwasserstoffflusses 5, enthaltend einen aromatischen Kohlenwasserstoff, nach einer der folgenden vier Arten und Reaktion:
d1) Durchführen einer nicht genauen Rektifizierung mit dem Flüssigphasen-Kohlenwasserstofffluss 5, der einen aromatischen Kohlenwasserstoff enthält, durch eine Trenneinheit 3, unter Erhalt eines Kohlenwasserstoffflusses 7 aus aromatischen Kohlenwasserstoffen mit gleich oder weniger als 7 Kohlenstoffatomen und eines Kohlenwasserstoffflusses 8 aus aromatischen Kohlenwasserstoffen mit mehr als 7 Kohlenstoffatomen und Trennen des Kohlenwasserstoffflusses 8 durch eine Trenneinheit 4, unter Erhalt eines Kohlenwasserstoffflusses 9, eines Flusses 10, der C₈-aromatischen Kohlenwasserstoff enthält, und eines C₉⁺-aromatischen Kohlenwasserstoffflusses 11, und Reaktion des Flusses 11 in einem Dealkylierungsreaktor, unter Erhalt eines C₈-aromatischen Kohlenwasserstoffflusses 201,
Erhalt eines Kohlenwasserstoffflusses 2 aus aromatischen Kohlenwasserstoffen mit gleich oder weniger als 7 Kohlenstoffatomen von dem Kohlenwasserstofffluss 7 und eines Teils oder allem von dem C₂⁺-Kohlenwasserstofffluss 6, worin der Kohlenwasserstofffluss 2 weiterhin wahlweise einen Teil oder alles von zumindest einem enthält, ausgewählt aus dem Kohlenwasserstofffluss 9 und einem C₂⁺-Kohlenwasserstofffluss 101 außerhalb des Reaktions-Trennsystems, und Rückführen des Kohlenwasserstoffflusses 2 zum Oxygenatfluss 1 zur weiteren Reaktion,
d2) Durchführen einer nicht-präzisen Rektifizierung mit dem Flüssigphasen-Kohlenwasserstofffluss 5, der einen aromatischen Kohlenwasserstoff enthält, durch eine Trenneinheit 3, unter Erhalt eines Kohlenwasserstoffflusses 7 aus aromatischen Kohlenwasserstoffen mit gleich oder weniger als 7 Kohlenstoffatomen und eines Kohlenwasserstoffflusses 8 aus aromatischen Kohlenwasserstoffen mit mehr als 7 Kohlenstoffatomen, und Erhalt eines Kohlenwasserstoffflusses 9, der nicht-aromatische Kohlenwasserstoffe enthält, eines Flusses 10, der C₈-aromatische Kohlenwasserstoffe enthält, und eines C₉⁺-aromatischen Kohlenwasserstoffflusses 11 von dem Kohlenwasserstofffluss 8 durch eine Trenneinheit 4,
Erhalt eines Kohlenwasserstoffflusses 15, der Dimethylbenzol enthält, von einem aromatischen C₉⁺-aromatischen Kohlenwasserstofffluss 12 und eines Methylbenzolflusses 13 außerhalb des Reaktions-Trennsystems durch einen Transalkylierungsreaktor, worin der C₉⁺-aromatische Kohlenwasserstofffluss 12 ausgewählt ist aus einem Teil oder allem von einem C₉⁺-aromatischen Kohlenwasserstofffluss 11 oder einer Mischung aus einem Teil oder allem von dem C₉⁺-aromatischen Kohlenwasserstofffluss 11 und einem C₉⁺-aromatischen Kohlenwasserstofffluss 106 außerhalb des Reaktions-Trennsystems,
Erhalt eines Kohlenwasserstoffflusses 2 aus aromatischem Kohlenwasserstoff mit gleich oder weniger als 7 Kohlenstoffatomen von dem Kohlenwasserstofffluss 7 und eines Teils oder von allem eines C₂⁺-Kohlenwasserstoffflusses 6, worin der Kohlenwasserstofffluss 2 weiterhin wahlweise einen Teil oder alles von zumindest einem enthält, ausgewählt aus dem Kohlenwasserstofffluss 9 und einem C₂⁺-Kohlenwasserstofffluss 101, außerhalb des Reaktions-Trennsystems, und Rückführen des Kohlenwasserstoffflusses 2 zu dem Oxygenatfluss 1 für die weitere Reaktion,
d3) Durchführen einer nicht-präzisen Rektifizierung mit dem Flüssigphasen-Kohlenwasserstofffluss 5, enthaltend einen aromatischen Kohlenwasserstoff, durch eine Trenneinheit 5, unter Erhalt eines Kohlenwasserstoffflusses 21 aus aromatischem Kohlenwasserstoff mit gleich oder weniger als 6 Kohlenstoffatomen und eines Kohlenwasserstoffflusses 22 aus aromatischem Kohlenwasserstoff mit mehr als 6 Kohlenstoffatomen, und Erhalt eines Kohlenwasserstoffflusses 23, enthaltend nicht-aromatische Kohlenwasserstoffe, eines Methylbenzolflusses 24, eines Flusses 25, enthaltend C₈-aromatische Kohlenwasserstoffe, und eines C₉⁺-aromatischen Kohlenwasserstoffflusses 26 von dem Fluss 22 durch eine Trenneinheit 6,
Erhalt eines Kohlenwasserstoffflusses 2 aus aromatischen Kohlenwasserstoffen mit gleich oder weniger als 7 Kohlenstoffatomen von einem Teil oder allen von dem Kohlenwasserstofffluss 6 und dem Kohlenwasserstofffluss 21, worin der Kohlenwasserstofffluss 2 weiterhin wahlweise einen Teil oder alles von zumindest einem enthält, ausgewählt aus dem Kohlenwasserstofffluss 23 und einem C₂⁺-Kohlenwasserstofffluss 101 außerhalb des Reaktions-Trennsystems, und Rückführen des Kohlenwasserstoffflusses 2 zu dem Oxygenatfluss 1 für die weitere Reaktion,
d4) Durchführen einer nicht-präzisen Rektifizierung mit dem Flüssigphasen-Kohlenwasserstofffluss 5, enthaltend einen aromatischen Kohlenwasserstoff, durch eine Trenneinheit 5, unter Erhalt eines Kohlenwasserstoffflusses 21 aus aromatischen Kohlenwasserstoffen mit gleich oder weniger als 6 Kohlenstoffatomen und eines Kohlenwasserstoffflusses 22 aus aromatischen Kohlenwasserstoffen mit mehr als 6 Kohlenstoffatomen, und Erhalt eines Kohlenwasserstoffflusses 23 mit nicht-aromatischen Kohlenwasserstoffen, eines Methylbenzolflusses 24, eines Flusses 25 mit C₈-aromatischen Kohlenwasserstoffen und eines C₉⁺-aromatischen Kohlenwasserstoffflusses 26 von dem Fluss 22 durch eine Trenneinheit 6,
Kontaktieren eines Methylbenzolflusses 27 und eines C₉⁺-aromatischen Kohlenwasserstoffflusses 28 mit einem Katalysator in einem Transalkylierungsreaktor, unter Erhalt eines Flusses 29, enthaltend Dimethylbenzol, worin der Fluss 27 ausgewählt ist aus einem Teil oder allem von dem Methylbenzolfluss 24 oder einem gemischten Fluss aus einem Teil oder allem von dem Fluss 24 und einem Methylbenzolfluss 105, außerhalb des Reaktions-Trennsystems, und der C₉⁺-aromatische Kohlenwasserstofffluss 28 ausgewählt ist aus einem von Teil oder allem von dem Fluss 26 oder einem gemischten Fluss aus einem Teil oder allem von dem Fluss 26 und einem C₉⁺-aromatischen Kohlenwasserstofffluss 106 außerhalb des Reaktions-Trennsystems,
Erhalt eines Kohlenwasserstoffflusses 2 aus aromatischen Kohlenwasserstoffen mit gleich oder weniger als 7 Kohlenstoffatomen von einem Teil oder allem von dem Kohlenwasserstofffluss 6 und des Kohlenwasserstoffflusses 21, worin der Kohlenwasserstofffluss 2 weiterhin wahlweise einen Teil oder alles von zumindest einem, ausgewählt aus dem Fluss 23 und einem C₂⁺-Kohlenwasserstofffluss 101 enthält, außerhalb des Reaktions-Trennsystems und Rückführen des Kohlenwasserstoffflusses 2 zu dem Oxygenatfluss 1 für die weitere Reaktion.

6. Verfahren zur Erzeugung eines aromatischen Kohlenwasserstoffs mit einem Oxygenat als Ausgangsmaterial gemäß einem der Ansprüche 1 bis 4, enthaltend zwei Aromatisierungsreaktoren, wobei das Verfahren die folgenden Schritte enthält:
h) unter den Prozessbedingungen einer Temperatur von 400 bis 550°C, eines Reaktionsdrucks von 0,01 bis 2,0 MPa und einer Gewicht- Raumgeschwindigkeit des/der Ausgangsmaterialien von 0,1 bis 4 h⁻¹ wird ein Fluss 1 aus Oxygenat(en) mit einem Katalysator für die Reaktion in einem ersten Aromatisierungsreaktor kontaktiert, unter Erhalt eines Kohlenwasserstoffflusses 3,
i) Entfernen von CO₂ und eines Teils von saurem Oxygenat, unter Erhalt eines Gasphasen-nicht-aromatischen Kohlenwasserstoffflusses 4, eines Flüssigphasen-Kohlenwasserstoffflusses 5, der aromatische Kohlenwasserstoffe enthält, und einer wässrigen Phase von einem Fluss 3 durch eine Trenneinheit 1,
j) Entfernen von Gas, CH₄, und eines Teils von Oxygenat(en) von dem Kohlenwasserstofffluss 31 durch eine Trenneinheit 2, unter Erhalt eines C₂⁺-nicht-aromatischen Kohlenwasserstoffflusses 32, worin der Kohlenwasserstofffluss 31 ein gemischter Kohlenwasserstofffluss aus dem Fluss 4 und dem Fluss 35 ist,
k) unter den Prozessbedingungen einer Temperatur von 450 bis 650°C, eines Reaktionsdrucks von 0,01 bis 2,0 MPa und einer Gewicht- Raumgeschwindigkeit von Ausgangsmaterial(ien) von 0,1 bis 4 h⁻¹ Kontaktieren eines Kohlenwasserstoffflusses 33 mit einem Katalysator in einem zweiten Aromatisierungsreaktor, unter Erhalt eines Kohlenwasserstoffflusses 34, worin der Kohlenwasserstofffluss 33 ausgewählt ist aus einem gemischten Kohlenwasserstofffluss aus dem Fluss 32 und einem Fluss I, worin der Fluss I ausgewählt ist aus einem Teil oder allem von zumindest einem von einem C₂⁺-nicht-aromatischen Kohlenwasserstofffluss 102 außerhalb des Reaktions-Trennsystems und einem Fluss 39,
1) Entfernen von CO₂ und einem Teil von saurem Oxygenat, unter Erhalt eines Gasphasen-nicht-aromatischen Kohlenwasserstoffflusses 35, eines Flüssigphasen-Kohlenwasserstoffflusses 36, enthaltend aromatische Kohlenwasserstoffe, und einer wässrigen Phase aus dem Kohlenwasserstofffluss 34 durch eine Trenneinheit 7,
m) weiteres Trennen des Flusses 5 und des Flusses 36 gemäß einer der folgenden vier Arten und Reaktion:
m1) Trennen des Flusses 5 und des Flusses 36 in einer Trenneinheit 8 durch eine nicht-präzise Rektifizierung, unter Erhalt eines Kohlenwasserstoffflusses 37 aus aromatischen Kohlenwasserstoffen mit gleich oder weniger als 7 Kohlenstoffatomen und eines Kohlenwasserstoffflusses 38 aus aromatischen Kohlenwasserstoffen mit mehr als 7 Kohlenstoffatomen und Trennen des Flusses 38 durch eine Trenneinheit 9, unter Erhalt des Flusses 39, enthaltend nicht-aromatische Kohlenwasserstoffe, eines C₈-aromatischen Kohlenwasserstoffflusses 40 und eines C₉⁺-aromatischen Kohlenwasserstoffflusses 41 und Reaktion des C₉⁺-aromatischen Kohlenwasserstoffflusses 41 oder eines gemischten Kohlenwasserstoffflusses des C₉⁺-aromatischen Kohlenwasserstoffflusses 41 und wahlweise eines C₉⁺-aromatischen Kohlenwasserstoffflusses 106 in einem Dealkylierungsreaktor, unter Erhalt eines C₈-aromatischen Kohlenwasserstoffflusses 202,
Erhalt eines Kohlenwasserstoffflusses 2 aus aromatischen Kohlenwasserstoffen mit gleich oder weniger als 7 Kohlenstoffatomen von dem Kohlenwasserstofffluss 37 und eines Flusses H, worin der Fluss H ausgewählt ist aus zumindest einem von einem C₂⁺-Kohlenwasserstofffluss 101 außerhalb des Reaktions-Trennsystems und des Flusses 39, und Rückführen des Kohlenwasserstoffflusses 2 zum Oxygenatfluss 1 für die weiter Reaktion,
m2) Trennen des Flusses 5 und des Flusses 36 in einer Trenneinheit 8 durch eine nicht-präzise Rektifizierung, unter Erhalt eines Kohlenwasserstoffflusses 37 von aromatischen Kohlenwasserstoffen mit gleich oder weniger als 7 Kohlenstoffatomen und eines Kohlenwasserstoffflusses 38 mit mehr als 7 Kohlenstoffatomen und Trennen des Flusses 38 durch eine Trenneinheit 9, unter Erhalt des Flusses 39 mit nicht-aromatischen Kohlenwasserstoffen, eines C₈-aromatischen Kohlenwasserstoffflusses 40 und eines C₉⁺-aromatischen Kohlenwasserstoffflusses 41,
Erhalt eines Flusses 44, enthaltend C₈-aromatische Kohlenwasserstoffe, von einem C₉⁺-aromatischen Kohlenwasserstofffluss 42 und eines Methylbenzolflusses 43 außerhalb eines Reaktions-Trennsystems, worin der C₉⁺-aromatische Kohlenwasserstofffluss 42 ausgewählt ist aus dem Fluss 41 oder einem gemischten Fluss aus dem Fluss 41 und einem C₉⁺-aromatischen Kohlenwasserstofffluss 106 außerhalb des Reaktions-Trennsystems,
Erhalt eines Kohlenwasserstoffflusses 2 aus aromatischen Kohlenwasserstoffen mit gleich oder weniger als 7 Kohlenstoffatomen von dem Kohlenwasserstofffluss 37 und eines Flusses H, worin der Fluss H ausgewählt ist aus zumindest einem von einem C₂⁺-Kohlenwasserstofffluss 101 außerhalb des Reaktions-Trennsystems und dem Fluss 39 und Rückführen des Kohlenwasserstoffflusses 2 zum Oxygenatfluss 1 für die weiter Reaktion,
m3) Trennen des Flusses 5 und des Flusses 36 in einer Trenneinheit 10 durch eine nicht-präzise Rektifizierung, unter Erhalt eines Kohlenwasserstoffflusses 47 aus aromatischen Kohlenwasserstoffen mit gleich oder weniger als 7 Kohlenstoffatomen und eines Kohlenwasserstoffflusses 48 mit mehr als 7 Kohlenstoffatomen, und Trennen des Flusses 48 durch eine Trenneinheit 11, unter Erhalt des Flusses 49 mit nicht-aromatischen Kohlenwasserstoffen, eines C₈-aromatischen Kohlenwasserstoffflusses 50 und eines C₉⁺-aromatischen Kohlenwasserstoffflusses 51,
Erhalt eines Flusses 2 aus aromatischen Kohlenwasserstoffen mit gleich oder weniger als 7 Kohlenstoffatomen von dem Fluss 47 und eines Flusses H, worin der Fluss H ausgewählt ist aus zumindest einem von C₂⁺-Kohlenwasserstofffluss 101 außerhalb des Reaktions-Trennsystems und dem Fluss 49 und Rückführen des Kohlenwasserstoffflusses 2 zum Oxygenatfluss 1 für die weiter Reaktion,
m4) Trennen des Flusses 5 und des Flusses 36 in einer Trenneinheit 9 durch eine nicht-präzise Rektifizierung, unter Erhalt eines Kohlenwasserstoffflusses 47 aus aromatischen Kohlenwasserstoffen mit gleich oder weniger als 7 Kohlenstoffatomen und eines Kohlenwasserstoffflusses 48 mit mehr als 7 Kohlenstoffatomen und Trennen des Flusses 48 durch eine Trenneinheit 9, unter Erhalt des Flusses 49 mit nicht-aromatischen Kohlenwasserstoffen, eines C₈-aromatischen Kohlenwasserstoffflusses 50 und eines C₉⁺-aromatischen Kohlenwasserstoffflusses 51,
Erhalt eines Flusses 54 aus aromatischen C₈-Kohlenwasserstoffen von einem C₉⁺-aromatischen Kohlenwasserstofffluss 52 und einem Methylbenzolfluss 53 außerhalb des Reaktions-Trennsystems, worin der C₉⁺-aromatische Kohlenwasserstofffluss 52 ausgewählt ist aus dem Fluss 51 oder einem gemischten Fluss aus dem Fluss 51 und einem C₉⁺-aromatischen Kohlenwasserstofffluss 106 außerhalb des Reaktions-Trennsystems,
Erhalt eines Kohlenwasserstoffflusses 2 aus aromatischen Kohlenwasserstoffen mit gleich oder weniger als 7 Kohlenstoffatomen von dem Fluss 47 und einem Fluss H, worin der Fluss H ausgewählt ist aus zumindest einem von C₂⁺-Kohlenwasserstofffluss 101 außerhalb des Reaktions-Trennsystems oder dem Fluss 49, und Rückführen des Kohlenwasserstoffflusses 2 zum Oxygenatfluss 1 für die weitere Reaktion.

7. Verfahren zur Erzeugung eines aromatischen Kohlenwasserstoffs mit einem Oxygenat als Ausgangsmaterial gemäß einem der Ansprüche 1 bis 6, **dadurch gekennzeichnet, dass** der Aromatisierungsreaktor zumindest einer von einem Fließbettreaktor, Festbettreaktor oder Bewegbettreaktor sein kann und der Transalkylierungsreaktor und Dealkylierungsreaktor Festbettreaktoren sind.

8. Verfahren zur Erzeugung eines aromatischen Kohlenwasserstoffs mit einem Oxygenat als Ausgangsmaterial gemäß einem der Ansprüche 1 bis 7, **dadurch gekennzeichnet, dass** der Aromatisierungsreaktor ein Fließbettreaktor und ein Bewegbettreaktor ist, wahlweise mit verschiedenen Regenerationssystemen oder mit einem gemeinsamen Regenerationssystem.

9. Verfahren zur Erzeugung eines aromatischen Kohlenwasserstoffs mit einem Oxygenat als Ausgangsmaterial gemäß einem der Ansprüche 1 bis 8, **dadurch gekennzeichnet, dass** der Aromatisierungskatalysator zumindest eine Molekularsieb-Aktivkomponente enthält, ausgewählt aus ZSM-5- und ZSM-11-Molekularsieben, worin das molare Verhältnis von Siliciumdioxid zu Aluminiumoxid im Molekularsieb von 10:1 bis 200:1 ist.

10. Verfahren zur Erzeugung eines aromatischen Kohlenwasserstoffs mit einem Oxygenat als Ausgangsmaterial gemäß einem der Ansprüche 1 bis 8, **dadurch gekennzeichnet, dass** der Transalkylierungskatalysator zumindest eine Molekularsieb-Aktivkomponente enthält, ausgewählt aus MOR-, ZSM-5- und BETA-Molekularsieben und das Dealkylierungsverfahren frei von Katalysator sein kann oder Oxid, einen Dealkylierungskatalysator vom Molekularsieb-Typ enthält.

11. Verfahren zur Erzeugung eines aromatischen Kohlenwasserstoffs mit einem Oxygenat als Ausgangsmaterial gemäß einem der Ansprüche 1 bis 10, **dadurch gekennzeichnet, dass** die Molekularsiebkomponente vor dem Formen des Katalysators oder der Katalysator, der die Modifizierungskomponente trägt, einer Hochtemperatur-hydrothermischen Behandlung unter den Bedingungen einer Temperatur von 400 bis 750°C, eines Partialdrucks von Wasserdampf von 5 bis 100 %, einer Behandlungszeit von 0,5 bis 120 Stunden unterworfen wird.

## Revendications

1. Procédé de production d'un hydrocarbure aromatique avec un oxygénat en tant que matière première, comprenant
i) la réaction d'un oxygénat dans au moins un réacteur d'aromatisation pour obtenir un produit de réaction d'aromatisation ;
ii) la séparation du produit de réaction d'aromatisation à travers une unité de séparation A pour obtenir un flux d'hydrocarbures en phase gazeuse X et un flux d'hydrocarbures en phase liquide Y ;
iii) après élimination du gaz et/ou d'une partie de l'oxygénat à partir du flux d'hydrocarbures en phase gazeuse X à travers une unité de séparation B, une réaction est réalisée dans un autre réacteur d'aromatisation et une séparation est réalisée à travers une unité de séparation A, pour obtenir un flux X2 contenant un hydrocarbure non aromatique et un flux X3 contenant un hydrocarbure aromatique ;
iv) un flux d'hydrocarbures mixte M d'un hydrocarbure aromatique présentant un nombre de carbones inférieur ou égal à 7 et un flux N des hydrocarbures résiduels est obtenu par une rectification non précise du flux d'hydrocarbures en phase liquide Y dans une unité de séparation C, facultativement après combinaison dudit flux Y audit flux X3 ;
v) la séparation du flux N des hydrocarbures résiduels à travers une unité de séparation D, pour obtenir un flux K contenant un hydrocarbure non aromatique, un flux J d'hydrocarbure aromatique en Cg et un flux L d'hydrocarbure aromatique en C₉⁺;
vi) le renvoi
a) du flux X2 contenant un hydrocarbure non aromatique, et
b) du flux d'hydrocarbures mixte M d'un hydrocarbure aromatique présentant un nombre de carbones inférieur ou égal à 7 et/ou d'une partie ou de la totalité du flux K contenant un hydrocarbure non aromatique, et facultativement
c) d'un flux d'hydrocarbures en C₂⁺ supplémentaire,
vers l'oxygénat figurant ci-dessus ; ou
le renvoi
a) du flux X2 contenant un hydrocarbure non aromatique, et
b) d'un flux d'hydrocarbures mixte M d'un hydrocarbure aromatique présentant un nombre de carbones inférieur ou égal à 7 et/ou d'une partie ou de la totalité du flux K contenant un hydrocarbure non aromatique,
vers le réacteur d'aromatisation en iii) ;
vii) facultativement, la réaction du flux L d'hydrocarbures aromatiques en C₉⁺ dans au moins un réacteur choisi parmi un réacteur de transalkylation et un réacteur de désalkylation pour obtenir un flux L1 d'hydrocarbure aromatique en Cg.

2. Procédé de production d'un hydrocarbure aromatique avec un oxygénat en tant que matière première selon la revendication 1, dans lequel l'étape iii) comprend une adsorption modulée en pression, une rectification et/ou une adsorption dans l'unité de séparation B et comprend des unités d'opération choisies parmi une extinction, un lavage alcalin et/ou un lavage à l'eau dans l'unité de séparation A, et
dans lequel l'étape v) comprend au moins l'une parmi une rectification et une rectification par extraction au solvant dans l'unité de séparation D.

3. Procédé de production d'un hydrocarbure aromatique avec un oxygénat en tant que matière première selon la revendication 1, dans lequel le flux d'hydrocarbures en phase liquide Y est séparé par les deux manières suivantes :
1) le flux Y entre dans une unité de séparation C1 et est séparé par une rectification non précise pour obtenir un flux d'hydrocarbures mixte M1 d'hydrocarbures aromatiques présentant un nombre de carbones inférieur ou égal à 6 et un flux d'hydrocarbures N1 présentant un nombre de carbones supérieur à 6, et le flux d'hydrocarbures N1 entre dans une unité de séparation D1 pour pouvoir obtenir un flux d'hydrocarbure aromatique en Cg et un flux d'hydrocarbure aromatique en C₉⁺;
2) le flux Y entre dans une unité de séparation C2 et est séparé par une rectification non précise pour obtenir un flux d'hydrocarbures mixte M2 d'hydrocarbures aromatiques présentant un nombre de carbones inférieur ou égal à 7 et un flux d'hydrocarbures N2 présentant un nombre de carbones supérieur à 7, et le flux d'hydrocarbures N2 entre dans une unité de séparation D2 pour pouvoir obtenir un flux d'hydrocarbure aromatique en Cg et un flux d'hydrocarbure aromatique en C₉⁺.

4. Procédé de production d'un hydrocarbure aromatique avec un oxygénat en tant que matière première selon l'une quelconque des revendications 1 à 3, dans lequel une partie ou la totalité du flux d'hydrocarbure non aromatique et du flux de l'oxygénat entrent en contact avec un catalyseur pour une réaction dans le même réacteur d'aromatisation ou en entrant dans des réacteurs d'aromatisation différents ; au moins un réacteur choisi dans le groupe se composant d'un réacteur de transalkylation et d'un réacteur de désalkylation dans ledit procédé est utilisé pour convertir un flux d'hydrocarbures aromatiques en C₉⁺ dans le produit d'hydrocarbures aromatiques en diméthylbenzène ; les conditions de réaction pour ledit réacteur de transalkylation sont une température allant de 350 à 550 °C, une pression de réaction allant de 0,1 à 5,0 MPa, un rapport molaire hydrogène/hydrocarbure allant de 1: 1 à 200: 1, une vitesse spatiale en poids de matière première allant de 0,1 à 15 h⁻¹ ; les conditions de réaction dudit réacteur de désalkylation sont une température de réaction allant de 300 à 800 °C, un rapport molaire hydrogène/hydrocarbure allant de 0,1 à 200: 1 et une vitesse spatiale en poids des hydrocarbures allant de 0,5 à 10 h⁻¹.

5. Procédé de production d'un hydrocarbure aromatique avec un oxygénat en tant que matière première selon l'une quelconque des revendications 1 à 4, comprenant un réacteur d'aromatisation, ledit procédé comprenant les étapes suivantes :
a) dans les conditions de traitement d'une température allant de 400 à 550 °C, une pression allant de 0,01 à 2,0 MPa et une vitesse spatiale en poids de matière(s) première(s) allant de 0,1 à 4 h⁻¹, la mise en contact d'un flux d'oxygénat(s) 1 avec un catalyseur pour une réaction dans un réacteur d'aromatisation afin d'obtenir un flux d'hydrocarbures 3 ;
b) l'élimination de CO₂ et d'une partie d'oxygénat(s) à partir dudit flux d'hydrocarbures 3 à travers une unité de séparation 1 pour obtenir un flux d'hydrocarbures non aromatiques en phase gazeuse 4, un flux d'hydrocarbures en phase liquide 5 contenant un hydrocarbure aromatique et une phase aqueuse ;
c) l'élimination de gaz, de CH₄ et d'une partie d'oxygénat(s) à partir dudit flux d'hydrocarbures non aromatiques en phase gazeuse 4 à travers une unité de séparation 2 pour obtenir un flux 6 d'hydrocarbures en C₂⁺;
d) la séparation supplémentaire du flux d'hydrocarbures en phase liquide 5 contenant un hydrocarbure aromatique selon l'une des quatre manières suivantes, et la réaction :
d1) la soumission du flux d'hydrocarbures en phase liquide 5 contenant un hydrocarbure aromatique à une rectification non précise à travers une unité de séparation 3 pour obtenir un flux d'hydrocarbure 7 d'hydrocarbures aromatiques présentant un nombre de carbones inférieur ou égal à 7, et un flux d'hydrocarbures 8 d'hydrocarbures aromatiques présentant un nombre de carbones supérieur à 7, et la séparation dudit flux d'hydrocarbures 8 à travers une unité de séparation 4 pour obtenir un flux d'hydrocarbures 9, un flux 10 contenant un hydrocarbure aromatique en Cg et un flux 11 d'hydrocarbure aromatique en C₉⁺, et la réaction dudit flux 11 dans un réacteur de désalkylation pour obtenir un flux 201 d'hydrocarbure aromatique en Cg ;
l'obtention d'un flux d'hydrocarbures 2 d'hydrocarbures aromatiques présentant un nombre de carbones inférieur ou égal à 7 à partir du flux d'hydrocarbures 7 et d'une partie ou de la totalité du flux 6 d'hydrocarbures en C₂⁺, dans lequel ledit flux d'hydrocarbures 2 comprend en outre facultativement une partie ou la totalité d'au moins l'un choisi parmi le flux d'hydrocarbures 9 et un flux 101 d'hydrocarbures en C₂⁺ à l'extérieur du système de réaction-séparation ; et le renvoi dudit flux d'hydrocarbures 2 vers le flux d'oxygénat(s) 1 pour une réaction supplémentaire ;
d2) la soumission du flux d'hydrocarbures en phase liquide 5 contenant un hydrocarbure aromatique à une rectification non précise à travers une unité de séparation 3 pour obtenir un flux d'hydrocarbures 7 d'hydrocarbures aromatiques présentant un nombre de carbones inférieur ou égal à 7 et un flux d'hydrocarbures 8 d'hydrocarbures aromatiques présentant un nombre de carbones supérieur à 7, et l'obtention d'un flux d'hydrocarbures 9 contenant des hydrocarbures non aromatiques, d'un flux 10 contenant des hydrocarbures aromatiques en Cg et d'un flux 11 d'hydrocarbure aromatique en C₉⁺ à partir dudit flux d'hydrocarbures 8 à travers une unité de séparation 4 ;
l'obtention d'un flux d'hydrocarbure 15 contenant du diméthylbenzène à partir d'un flux 12 d'hydrocarbure aromatique en C₉⁺ et d'un flux de méthylbenzène 13 à l'extérieur du système de réaction-séparation à travers un réacteur de transalkylation, dans lequel le flux 12 d'hydrocarbure aromatique en C₉⁺ est choisi parmi l'un d'une partie ou de la totalité d'un flux 11 d'hydrocarbure aromatique en C₉⁺ ou d'un mélange d'une partie ou de la totalité du flux 11 d'hydrocarbure aromatique en C₉⁺ et d'un flux 106 d'hydrocarbure aromatique en C₉⁺ à l'extérieur du système de réaction-séparation ;
l'obtention d'un flux d'hydrocarbures 2 d'hydrocarbure aromatique présentant un nombre de carbones inférieur ou égal à 7 à partir du flux d'hydrocarbures 7 et d'une partie ou de la totalité d'un flux 6 d'hydrocarbures en C₂⁺, dans lequel ledit flux d'hydrocarbures 2 comprend en outre facultativement une partie ou la totalité d'au moins l'un choisi parmi le flux d'hydrocarbures 9 et un flux 101 d'hydrocarbures en C₂⁺ à l'extérieur du système de réaction-séparation ; et le renvoi dudit flux d'hydrocarbures 2 vers le flux d'oxygénat(s) 1 pour une réaction supplémentaire ;
d3) la soumission du flux d'hydrocarbures en phase liquide 5 contenant un hydrocarbure aromatique à une rectification non précise à travers une unité de séparation 5 pour obtenir un flux d'hydrocarbure 21 d'hydrocarbure aromatique présentant un nombre de carbones inférieur ou égal à 6 et un flux d'hydrocarbures 22 d'hydrocarbure aromatique présentant un nombre de carbones supérieur à 6, et l'obtention d'un flux d'hydrocarbures 23 contenant des hydrocarbures non aromatiques, d'un flux de méthylbenzène 24, d'un flux 25 contenant des hydrocarbures aromatiques en Cg et d'un flux 26 d'hydrocarbure aromatique en C₉⁺ à partir dudit flux 22 à travers une unité de séparation 6 ;
l'obtention d'un flux d'hydrocarbures 2 d'hydrocarbures aromatiques présentant un nombre de carbones inférieur ou égal à 7 à partir d'une partie ou de la totalité du flux d'hydrocarbures 6 et du flux d'hydrocarbures 21, dans lequel ledit flux d'hydrocarbures 2 comprend en outre facultativement une partie ou la totalité d'au moins l'un choisi parmi le flux d'hydrocarbures 23 et un flux 101 d'hydrocarbures en C₂⁺ à l'extérieur du système de réaction-séparation ; et le renvoi dudit flux d'hydrocarbures 2 vers le flux d'oxygénat 1 pour une réaction supplémentaire ;
d4) la soumission du flux d'hydrocarbures en phase liquide 5 contenant un hydrocarbure aromatique à une rectification non précise à travers une unité de séparation 5 pour obtenir un flux d'hydrocarbures 21 d'hydrocarbures aromatiques présentant un nombre de carbones inférieur ou égal à 6 et un flux d'hydrocarbures 22 d'hydrocarbures aromatiques présentant un nombre de carbones supérieur à 6, et l'obtention d'un flux d'hydrocarbures 23 contenant des hydrocarbures non aromatiques, d'un flux de méthylbenzène 24, d'un flux 25 contenant des hydrocarbures aromatiques en Cg et d'un flux 26 d'hydrocarbure aromatique en C₉⁺ à partir dudit flux 22 à travers une unité de séparation 6 ;
la mise en contact d'un flux de méthylbenzène 27 et d'un flux 28 d'hydrocarbure aromatique en C₉⁺ avec un catalyseur dans un réacteur de transalkylation pour obtenir un flux 29 contenant du diméthylbenzène, dans lequel ledit flux 27 est choisi parmi l'un d'une partie ou de la totalité du flux de méthylbenzène 24 ou d'un flux mixte d'une partie ou de la totalité du flux 24 et d'un flux de méthylbenzène 105 à l'extérieur du système de réaction-séparation, et ledit flux 28 d'hydrocarbure aromatique en C₉⁺ est choisi parmi l'un d'une partie ou de la totalité du flux 26 ou d'un flux mixte d'une partie ou de la totalité du flux 26 et d'un flux 106 d'hydrocarbure aromatique en C₉⁺ à l'extérieur du système de réaction-séparation ;
l'obtention d'un flux d'hydrocarbures 2 d'hydrocarbures aromatiques présentant un nombre de carbones inférieur ou égal à 7 à partir d'une partie ou de la totalité du flux d'hydrocarbure 6 et du flux d'hydrocarbures 21, dans lequel ledit flux d'hydrocarbures 2 comprend en outre facultativement une partie ou la totalité d'au moins l'un choisi parmi le flux 23 et un flux 101 d'hydrocarbures en C₂⁺ à l'extérieur du système de réaction-séparation ; et le renvoi dudit flux d'hydrocarbures 2 vers le flux d'oxygénat(s) 1 pour une réaction supplémentaire.

6. Procédé de production d'un hydrocarbure aromatique avec un oxygénat en tant que matière première selon l'une quelconque des revendications 1 à 4, comprenant deux réacteurs d'aromatisation, ledit procédé comprenant les étapes suivantes :
h) dans les conditions de traitement d'une température allant de 400 à 550 °C, d'une pression de réaction allant de 0,01 à 2,0 MPa et d'une vitesse spatiale en poids de matière(s) première(s) allant de 0,1 à 4 h⁻¹, la mise en contact d'un flux d'oxygénat(s) 1 avec un catalyseur pour une réaction dans un premier réacteur d'aromatisation afin d'obtenir un flux d'hydrocarbures 3 ;
i) l'élimination de CO₂ et d'une partie d'oxygénat acide pour obtenir un flux d'hydrocarbure non aromatique en phase gazeuse 4, un flux d'hydrocarbures en phase liquide 5 contenant des hydrocarbures aromatiques et une phase aqueuse à partir d'un flux 3 à travers une unité de séparation 1 ;
j) l'élimination de gaz, de CH₄ et d'une partie d'oxygénat(s) à partir dudit flux d'hydrocarbures 31 à travers une unité de séparation 2 pour obtenir un flux 32 d'hydrocarbures non aromatiques en C₂⁺, dans lequel le flux d'hydrocarbures 31 est un flux d'hydrocarbures mixte du flux 4 et du flux 35 ;
k) dans les conditions de traitement d'une température allant de 450 à 650 °C, d'une pression de réaction allant de 0,01 à 2,0 MPa et d'une vitesse spatiale en poids de matière(s) première(s) allant de 0,1 à 4 h¹, la mise en contact d'un flux d'hydrocarbures 33 avec un catalyseur dans un deuxième réacteur d'aromatisation pour obtenir un flux d'hydrocarbures 34, dans lequel ledit flux d'hydrocarbures 33 est choisi parmi un flux d'hydrocarbures mixte du flux 32 et d'un flux I, dans lequel le flux I est choisi parmi une partie ou la totalité d'au moins l'un d'un flux 102 d'hydrocarbure non aromatique en C₂⁺ à l'extérieur du système de réaction-séparation et d'un flux 39 ;
I) l'élimination de CO₂ et d'une partie d'oxygénat acide pour obtenir un flux d'hydrocarbure non aromatique en phase gazeuse 35, un flux d'hydrocarbures en phase liquide 36 contenant des hydrocarbures aromatiques et une phase aqueuse à partir du flux d'hydrocarbures 34 à travers une unité de séparation 7 ;
m) la séparation supplémentaire du flux 5 et du flux 36 selon l'une des quatre manières suivantes, et la réaction :
m1) la séparation du flux 5 et du flux 36 dans une unité de séparation 8 par une rectification non précise pour obtenir un flux d'hydrocarbures 37 d'hydrocarbures aromatiques présentant un nombre de carbones inférieur ou égal à 7 et un flux d'hydrocarbures 38 d'hydrocarbures aromatiques présentant un nombre de carbones supérieur à 7, et la séparation dudit flux 38 à travers une unité de séparation 9 pour obtenir le flux 39 contenant des hydrocarbures non aromatiques, un flux 40 d'hydrocarbure aromatique en Cg et un flux 41 d'hydrocarbure aromatique en C₉⁺, et la réaction dudit flux 41 d'hydrocarbure aromatique en C₉⁺ ou d'un flux d'hydrocarbures mixte du flux 41 d'hydrocarbure aromatique en C₉⁺ et facultativement d'un flux 106 d'hydrocarbure aromatique en C₉⁺ dans un réacteur de désalkylation pour obtenir un flux 202 d'hydrocarbure aromatique en Cg ;
l'obtention d'un flux d'hydrocarbures 2 d'hydrocarbures aromatiques présentant un nombre de carbones inférieur ou égal à 7 à partir du flux d'hydrocarbures 37 et d'un flux H, dans lequel ledit flux H est choisi parmi au moins l'un d'un flux 101 d'hydrocarbures en C₂⁺ à l'extérieur du système de réaction-séparation et du flux 39 ; et le renvoi dudit flux d'hydrocarbures 2 vers le flux d'oxygénat(s) 1 pour une réaction supplémentaire ;
m2) la séparation du flux 5 et du flux 36 dans une unité de séparation 8 par une rectification non précise pour obtenir un flux d'hydrocarbures 37 d'hydrocarbures aromatiques présentant un nombre de carbones inférieur ou égal à 7 et un flux d'hydrocarbures 38 présentant un nombre de carbones supérieur à 7, et la séparation dudit flux 38 à travers une unité de séparation 9 pour obtenir le flux 39 contenant des hydrocarbures non aromatiques, un flux 40 d'hydrocarbure aromatique en Cg et un flux 41 d'hydrocarbure aromatique en C₉⁺,
l'obtention d'un flux 44 contenant des hydrocarbures aromatiques en Cg à partir d'un flux 42 d'hydrocarbure aromatique en C₉⁺ et d'un flux de méthylbenzène 43 à l'extérieur d'un système de réaction-séparation, dans lequel le flux 42 d'hydrocarbure aromatique en C₉⁺ est choisi parmi le flux 41 ou un flux mixte du flux 41 et d'un flux 106 d'hydrocarbure aromatique en C₉⁺ à l'extérieur du système de réaction-séparation,
l'obtention d'un flux d'hydrocarbures 2 d'hydrocarbures aromatiques présentant un nombre de carbones inférieur ou égal à 7 à partir du flux d'hydrocarbures 37 et d'un flux H, dans lequel ledit flux H est choisi parmi au moins l'un d'un flux 101 d'hydrocarbures en C₂⁺ à l'extérieur du système de réaction-séparation et du flux 39 ; et le renvoi dudit flux d'hydrocarbures 2 vers le flux d'oxygénat(s) 1 pour une réaction supplémentaire ;
m3) la séparation du flux 5 et du flux 36 dans une unité de séparation 10 par une rectification non précise pour obtenir un flux d'hydrocarbures 47 d'hydrocarbures aromatiques présentant un nombre de carbones inférieur ou égal à 7 et un flux d'hydrocarbures 48 présentant un nombre de carbones supérieur à 7, et la séparation dudit flux 48 à travers une unité de séparation 11 pour obtenir le flux 49 contenant des hydrocarbures non aromatiques, un flux 50 d'hydrocarbure aromatique en Cg et un flux 51 d'hydrocarbure aromatique en C₉⁺ ;
l'obtention d'un flux d'hydrocarbures 2 d'hydrocarbures aromatiques présentant un nombre de carbones inférieur ou égal à 7 à partir du flux 47 et d'un flux H, dans lequel ledit flux H est choisi parmi au moins l'un d'un flux 101 d'hydrocarbures en C₂⁺ à l'extérieur du système de réaction-séparation et du flux 49; et le renvoi dudit flux d'hydrocarbures 2 vers le flux d'oxygénat 1 pour une réaction supplémentaire ;
m4) la séparation du flux 5 et du flux 36 dans une unité de séparation 9 par une rectification non précise pour obtenir un flux d'hydrocarbures 47 d'hydrocarbures aromatiques présentant un nombre de carbones inférieur ou égal à 7 et un flux d'hydrocarbures 48 présentant un nombre de carbones supérieur à 7, et la séparation dudit flux 48 à travers une unité de séparation 9 pour obtenir un flux 49 contenant des hydrocarbures non aromatiques, un flux 50 d'hydrocarbure aromatique en Cg et un flux 51 d'hydrocarbure aromatique en C₉⁺,
l'obtention d'un flux 54 contenant des hydrocarbures aromatiques en Cg à partir d'un flux 52 d'hydrocarbure aromatique en C₉⁺ et d'un flux de méthylbenzène 53 à l'extérieur du système de réaction-séparation, dans lequel le flux 52 d'hydrocarbure aromatique en C₉⁺ est choisi parmi le flux 51 ou un flux mixte du flux 51 et d'un flux 106 d'hydrocarbure aromatique en C₉⁺ à l'extérieur du système de réaction-séparation,
l'obtention d'un flux d'hydrocarbures 2 d'hydrocarbures aromatiques présentant un nombre de carbones inférieur ou égal à 7 à partir du flux 47 et d'un flux H, dans lequel ledit flux H est choisi parmi au moins l'un du flux 101 d'hydrocarbure en C₂⁺ à l'extérieur du système de réaction-séparation ou du flux 49; et le renvoi dudit flux d'hydrocarbures 2 vers le flux d'oxygénat 1 pour une réaction supplémentaire.

7. Procédé de production d'un hydrocarbure aromatique avec un oxygénat en tant que matière première selon l'une quelconque des revendications 1 à 6, **caractérisé en ce que** ledit réacteur d'aromatisation peut être au moins l'un parmi un réacteur à lit fluidisé, un réacteur à lit fixe ou un réacteur à lit mobile, et ledit réacteur de transalkylation et ledit réacteur de désalkylation sont des réacteurs à lit fixe.

8. Procédé de production d'un hydrocarbure aromatique avec un oxygénat en tant que matière première selon l'une quelconque des revendications 1 à 7, **caractérisé en ce que** ledit réacteur d'aromatisation est un réacteur à lit fluidisé et un réacteur à lit mobile, facultativement avec des systèmes de régénération différents ou avec un système de régénération commun.

9. Procédé de production d'un hydrocarbure aromatique avec un oxygénat en tant que matière première selon l'une quelconque des revendications 1 à 8, **caractérisé en ce que** ledit catalyseur d'aromatisation comprend au moins un composant actif de tamis moléculaire choisi parmi des tamis moléculaires ZSM-5 et ZSM-11, dans lequel le rapport molaire de l'oxyde de silicium sur l'oxyde d'aluminium dans le tamis moléculaire va de 10:1 à 200: 1.

10. Procédé de production d'un hydrocarbure aromatique avec un oxygénat en tant que matière première selon l'une quelconque des revendications 1 à 8, **caractérisé en ce que** ledit catalyseur de ransalkylation comprend au moins un composant actif de tamis moléculaire choisi parmi des tamis moléculaires MOR, ZSM-5 et BETA, et ledit processus de désalkylation peut être dépourvu de catalyseur ou comprend un catalyseur de désalkylation de type tamis moléculaire d'oxyde.

11. Procédé de production d'un hydrocarbure aromatique avec un oxygénat en tant que matière première selon l'une quelconque des revendications 1 à 10, **caractérisé en ce que** le composant de tamis moléculaire avant le moulage du catalyseur ou le catalyseur supportant le composant de modification est soumis à un traitement hydrothermique à haute température dans les conditions d'une température allant de 400 à 750 °C, d'une pression partielle de vapeur d'eau allant de 5 à 100 %, d'une période de traitement allant de 0,5 à 120 heures.
